(19) Europäisches Patentamt
European Patent Office
Office européen des brevets

(11) Publication number: **0 033 433**
**A1**

(12) # EUROPEAN PATENT APPLICATION

(21) Application number: 80810346.9

(22) Date of filing: 10.11.80

(51) Int. Cl.³: C 07 H 17/08
C 07 H 15/22, A 61 K 31/70
C 12 P 19/62, C 12 P 19/48
//(C12P19/62, C12R1/29),
(C12P19/48, C12R1/29)

(30) Priority: 09.11.79 US 93080
21.12.79 US 106184
14.03.80 US 130330

(43) Date of publication of application:
12.08.81 Bulletin 81/32

(84) Designated Contracting States:
AT BE CH DE FR GB IT LI LU NL SE

(71) Applicant: SCHERING CORPORATION
2000 Galloping Hill Road
Kenilworth, New Jersey 07033(US)

(72) Inventor: Waitz, Jay Allan
Box 139 Rd. 1. Daly Road
Far Hills New Jersey 07931(US)

(72) Inventor: Reiblein, Walter Joseph
40 Verona Place
Verona New Jersey 07044(US)

(72) Inventor: Truumees, Imbi
40 Gilmore Avenue
Cresskill New Jersey 07626(US)

(72) Inventor: Ganguly, Ashit Kumar
96 Cooper Avenue Upper Montclair
Essex New Jersey 07043(US)

(72) Inventor: Liu, Yi-Tsung
19 Schluyer Street
Parsippany New Jersey 07054(US)

(72) Inventor: Sarre, Olga
12 Vahalla Way Verona
Essex New Jersey(US)

(72) Inventor: Jaret, Robert
52 North Mitchell Avenue
Livingston New Jersey 07039(US)

(72) Inventor: Schumacher, Doris
76 Edgewood Drive
Florham Park New Jersey 07932(US)

(74) Representative: Antony, Fritz, Dr. et al,
P.O. Box 601 Winkelriedstrasse 35
CH-6002 Lucerne(CH)

(54) **Macrolide antibiotics, process for their preparation, inter alia by fermentation, and pharmaceutical compositions containing them.**

(57) Disclosed are novel macrolide antibiotics of the general formula

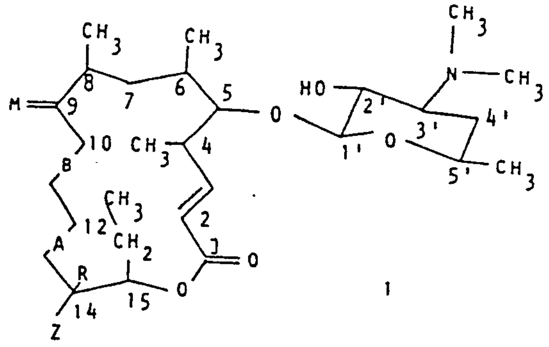

I

wherein R is H or OH; M is O or (H,OH); Z is the group of formula II,

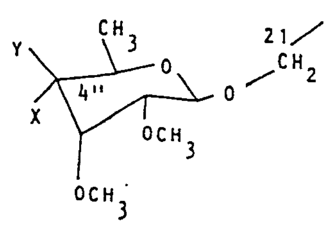

II

./...

EP 0 033 433 A1

$-CH_2OH$ or groups which may be derived therefrom; A represents a double bond between carbon atoms of positions 12 and 13 or, together with the carbon atoms of positions 12 and 13, an oxirane ring. B represents a double bond between the carbon atoms of positions 10 and 11 or together with the carbon atoms of positions 10 and 11 the group $-CH_2-CH(Q^1)-$ or $-CH_2-CH(Q^2)-$ and the non-toxic pharmaceutically acceptable ester and acid addition salts thereof.

The novel antibiotic complex designated antibiotic AR-5 of the formula

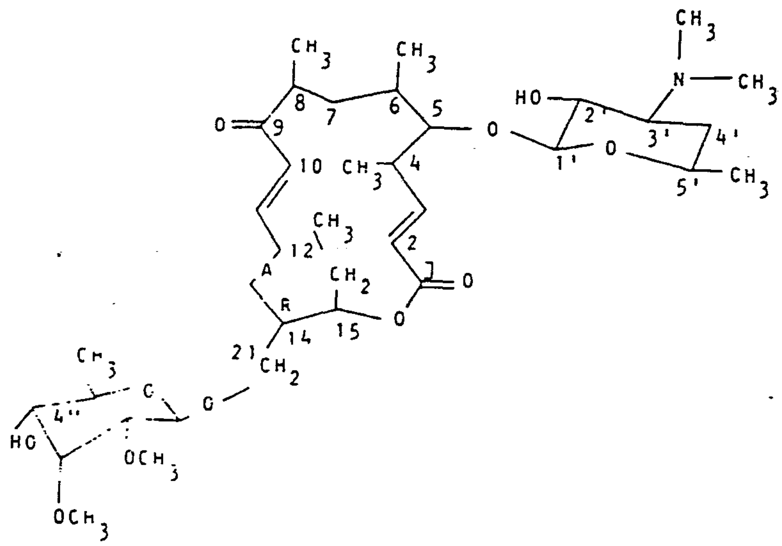

(falling within the scope of formula I), wherein R is hydrogen or hydroxy, and A represents a double bond or together with the carbon atoms of positions 12 and 13 an oxirane ring, can be produced by fermentation of a new species of the genus *Micromonospora*, which is *Micromonospora Polytrota* (e.g. NRRL 12066) The other compounds of formula I can be derived from the compounds of antibiotic AR-5 complex

The compounds shown antibacterial activity.

The gentamycine C complex is also produced by micromonospora polytrota NRRL 12066

 TITLE MODIFIED
see front page

MACROLIDE ANTIBIOTICS, PROCESS FOR THEIR PRODUCTION AND PHARMACEUTICAL COMPOSITIONS CONTAINING THEM.

This invention relates to novel antibiotically active substances, to processes for their preparation, and to compositions containing such substances. More specifically this invention relates to a novel antibiotic complex designated Antibiotic AR-5 and to its members, especially Antibiotic AR-5 component 1, Antibiotic AR-5 component 2, and derivatives thereof. Some of the novel antibiotically active substances can be obtained by the cultivation of a hitherto undescribed species of the genus Micromonospora. These substances can be transformed into their chemical derivatives. Gentamicin C, a known antibiotically active substance is coproduced by the fermentation of the hitherto undescribed microorganism.

The new macrolide antibiotics according to the present invention are compounds of the general formula I

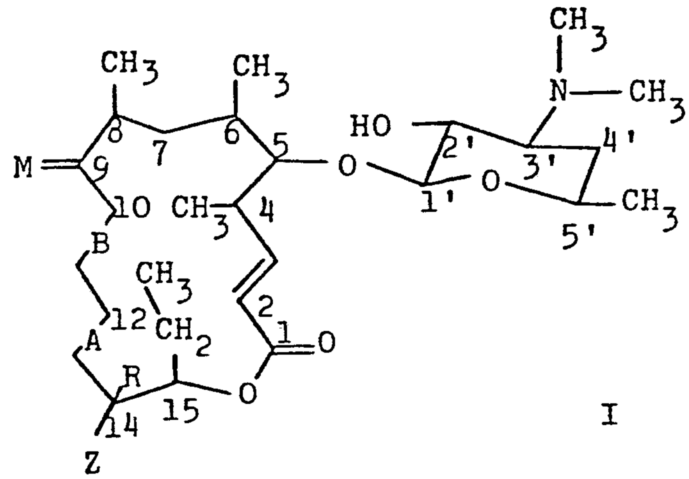

I

wherein R is H or OH; M is O or (H,OH); Z is the group of formula II

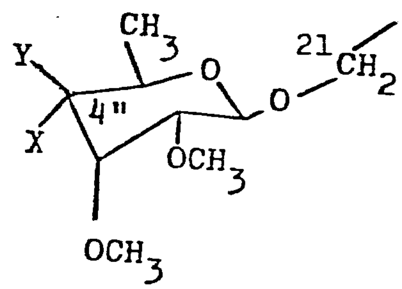

II

wherein X is hydrogen, OH, mesyloxy, tosyloxy, trifluoromethane sulfonyloxy, halo, azido, amino, isocyanato, isothiocyanato, guanidino, $-NHR^6$, $-NR^6R^7$, $-NHR^8$, $-NHC(O)OR^6$, $-NHC(O)OR^8$, $-NHC(NH)R^6$, $-NH(CH_2)pOH$ or $-NH(CH_2)pOR^{10}$, wherein p is an integer of from 2 to 18, or $-NHCOR^6$ when Y is hydrogen; or Y in combination with X is oxo, $=N-NH_2$, $=N-NHR^{11}$ or $=NR^{11}$;

A represents a double bond between the carbon atoms of positions 12 and 13 or, together with the carbon

atoms of positions 12 and 13, an oxirane ring; B represents a double bond between the carbon atoms of positions 10 and 11 or, provided R is H, M is O, X is OH, Y is H, and A together with the carbon atoms of positions 12 and 13 represents an oxirane ring, B may also be a single bond between the carbon atoms of positions 10 and 11, or, provided A together with the carbon atoms of positions 12 and 13 represents an oxirane ring and X is OH and Y is H, B together with the carbon atoms of positions 10 and 11 may also represent the group $-CH_2-CH(Q^1)-$ or $-CH_2-CH(Q^2)-$, wherein $Q^1$ is a nitrogen containing moiety and $Q^2$ is a sulfur containing moiety (the nitrogen or sulfur respectively being attached to the carbon atom of position 11), wherein $-Q^1$ is $-NH_2$, $-NHC(O)R^6$, $-NHR^6$, $-NR^6R^7$, $-NHR^{10}OH$, $-N=CHR^6$, $-NHC(S)NHR^6$, $-NHC(NH)R^6$, $-NHC(O)OR^6$, $-NHC(O)NHR^6$, $-NHC(NH)NH_2$, $-NH-C(O)-$ 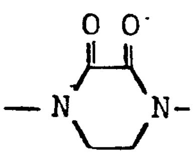 $-CH_2CH_3$, $-NHC(NH)NHR^6$, an amino acid residue, an esterified amino acid residue or a heterocyclic radical of 3 to 7 members containing either nitrogen as the only heteroatom or nitrogen and a second heteroatom which is nitrogen, oxygen, or sulfur;

$-Q^2$ is $-S-R^6$, thioxanthyl, $-S-R^{10}-NHR^6$, $-S-R^8$, $S-R^9$, $-S-R^{10}-R^9$, $-S-R^{10}-R^8-R^9$, $-S-R^8-R^9$, $-S-R^{10}-C(O)OR^6$, $-S-R^{10}-COOH$, a sulfur containing amino acid residue, or a sulfur containing esterified amino acid residue;

and wherein, provided A and B represent double bonds, Z may also be $-CH_2OH$, $-CHO$, $-CH_2NH_2$, $-CH_2NHR^2$, $-CH_2NR^2R^3$, $-CH_2OCOR^2$, $-CH_2NHCOR^2$, $-CH_2NR^3COR^2$, $-CH_2NH-C(=NH)-R^2$, $-CH_2NHC(=NH)-NHR^5$, $-CH_2NHCO-$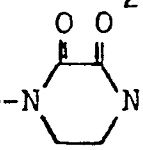$-CH_2CH_3$, $-CH_2O$-glycosyl excluding $-CH_2O$-mycinosyl and $-CH_2O$-(3"-desmethyl-mycinosyl), $-CH_2$-ureido or $-CH_2$-thioureido;

wherein $R^2$ and $R^3$ independent of each other represent an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen, or in the amino groups one of substituents $R^2$ and $R^3$ is hydrogen and the other is the group $-(CH_2)_mOR^4$ or -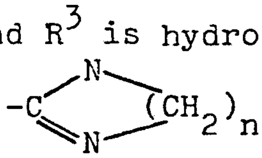, wherein m is an integer of from two to ten, n is an integer of from 2 to 6 and $R^4$ is an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen; and $R^5$ is hydrogen, $R^2$ or $-CH_2CH_2-$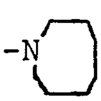 ; $R^6$ and $R^7$ independent of each other represent an alkyl group containing 1 to 18 carbon atoms, an aralkylgroup containing 7 to 18 carbon atoms; $R^8$ is an aryl group; $R^9$ is a heterocyclic group which contains 3 to 7 members, wherein the hetero atoms(s)

is (are) selected from sulfur, nitrogen and oxygen; and $R^{10}$ is an alkyl group containing 1 to 18 carbon atoms; $R^{11}$ represents an alkyl group containing 1 to 18 carbon atoms, an aralkyl group containing 7 to 18 carbon atoms or a heterocyclic group containing 3 to 7 members wherein the hetero atom(s) is(are) selected from sulfur, nitrogen and oxygen; and the non-toxic pharmaceutically acceptable esters thereof and the acid addition salts of the compounds of formula I and of their esters.

The novel antibiotic complex designated antibiotic AR-5 can be produced by fermentation of a new species of the genus Micromonospora as described below. This antibiotic complex contains four compounds of formula I: antibiotic AR-5 component 1(also referred to as antibiotic AR5-1) and antibiotic AR-5 component 2 (also referred to as antibiotic AR5-2), wherein M is oxo, Z is the group of formula II wherein X is hydroxy and Y is hydrogen, A together with the carbon atoms of positions 12 and 13 represents an oxirane ring and B represents a double bond between the carbon atoms of positions 10 and 11, and wherein R is hydrogen (antibiotic AR5-1) or hydroxy (antibiotic AR5-2), and the corresponding 12,13-desepoxy-12,13-dehydro derivatives (also referred to as 12,13-desepoxy-12,13-dehydro antibiotic AR5-1 and 12,13-desepoxy-12,13-dehydro antibiotic AR5-2).

In these compounds the group of formula II has the configuration of ⊢⊣ mycinose. The British patent publication 2020647, published November 21, 1979 appears to disclose antibiotic AR5-1, antibiotic AR5-2 and their 12,13-desepoxy-12,13-dehydro analogs. However, it is to be noted that the U.S. Application Ser. No. 93080 priority of which is claimed for the present application has been filed on November 9, 1979.

The novel antibiotic complex designated antibiotic AR-5 can be produced by fermentation of the novel species designated Micromonospora polytrota. The other compounds of formula I can be obtained from these compounds by chemical processes described further below. Compounds of formula I wherein M is (H,OH) and/or B is $-CH_2-CH(Q^1)-$ or $-CH_2-CH(Q^2)-$ and/or Y (when Z is the group of formula II) is hydrogen may exist in isomeric forms due to the fact that they contain at least one additional asymetric carbon atom in position 9 and/or 11 and/or 4". The invention includes all possible optically active forms and racemic mixtures of the compounds.

The microorganism was isolated from a soil sample collected in Campeche, Mexico. A culture of this microorganism has been made a part of the permanent collection of the Northern Utilization and Research Division, Agricultural Research Service, U.S. Department of Agriculture, where it has been assigned accession number NRRL 12066. Subcultures of Micromonospora polytrota NRRL 12066 are available to the public from the afore mentioned agency. A culture of this microorganism has been made a part of the collection of the

American Type Culture Collection (ATCC), where it has been assigned accession number ATCC 31584. Subcultures of Micromonospora polytrota ATCC 31584 are available to the public from the ATCC.

Micromonospora polytrota (sometimes referred to herein as M.polytrota) is aerobic and grows well on a variety of solid and liquid nutrient media. It exhibits especially good growth and antibiotic production under submerged aerobic conditions.

The microorganism may be distinguished from other heretofore known species of the genus Micromonospora by a variety of taxonomical parameters. For example, Micromonospora polytrota forms spores, grows on D- and L-arabinose and fructose; fails to grow on rhamnose and D-xylose; utilizes citrate, formate, lactate and oxalate; grows in the presence of 50 mcg per milliliter of gentamicin, sisomicin, kanamycin, lincomycin and clindamycin; exhibits sensitivity to rosaramicin and chloramphenicol; hydrolyzes hypoxanthine and hippurate; does not hydrolyze xylan and chitin.

The microorganism also forms urease and allantionase; survives a temperature of 50$^{\circ}$C for eight hours.

Other distinguishing characteristics which aid in determining that Micromonospora polytrota is novel are its growth characteristics on various descriptive media.

The foregoing distinguishing characteristics were determined using the following procedures whose results are set forth in detail in the disclosure that follows.

Strain Maintenance

The initial source material was a freeze-dried preparation. The contents of the vial were suspended in 10 ml of broth consisting of yeast extract, 5g; dextrose, 10 g; soluble starch, 20 g; NZ-Amine type A (Difco), 5g; $CaCO_3$, 1 g; tap water, 1000 ml; in 25 mm tubes stoppered with Morton closures. The pH was adjusted to 7.2 before autoclaving. The broth suspension was incubated at 30°C on a rotary shaker (New Brunswick, Model G-52) at 250 rpm for 3 to 4 days. After growth, 5 ml of the resulting biomass was transferred into 50 ml of fresh medium having the composition described above in a 250 ml Erlenmeyer flask stoppered with cotton. Cultures were incubated as described above and harvested after 3 to 4 days. Five ml aliquots of the resulting biomass were

asceptically dispensed into 17 x 60 mm sterile screw-capped vials and stored at -18°C.

Preparation of Inocula

One ml of thawed cell suspension was used to inoculate 25 mm tubes containing 10 ml of the strain maintenance broth, incubated as described above, and 5% inoculum transferred to two tubes of fresh broth. After three days at 30°C, the cells were harvested by centrifugation (Sorvall, Model GLC-1) at 450 x g for 15 minutes in 15 ml graduated tubes, washed twice with sterile distilled water, and resuspended in water to three times the packed cell volume. The resulting washed suspension was used as inoculum for the taxonomy tests reported below.

A 1% transfer of Micromonospora polytrota was made into duplicate 25 x 150 mm tubes containing 10 ml of a medium consisting of yeast extract, 1 g; soluble starch, 1 g; dextrose, 1 g; $CaCO_3$, 1 g; tap water , 1000 ml; or a medium consisting of yeast extract, 10 g; soluble starch, 20 g; $CaCO_3$, 1 g; tap water, 1000 ml; or a medium consisting of yeast extract, 5 g; dextrose, 10 g; $CaCO_3$; 1 g; tap water, 1000 ml; or a medium consisting of yeast extract, 1 g; dextrose, 10 g; $CaCO_3$, 1 g;

tap water, 1000 ml. Tubes were stoppered with Morton closures and incubated at 35°C on a rotary shaker (New Brunswick Scientific, Model G-52) at 250 rpm. At 3, 5, 7 and 14 days, aliquots from the tubes were thinly spread onto the surface of glass cover slips, air dried and inverted onto a glass slide into a drop of dilute crystal violet solution (1:10 with distilled water). Slides were examined under the phase microscope at magnifications up to 2000 times.

Micromonospora polytrota was inoculated onto the surface of various agar media in petri dishes: an agar consisting of yeast extract, 10 g; dextrose, 10 g; agar, 15 g; tap water, 1000 ml; pH 7.0; a water agar consisting of tap water, 1000 ml; agar, 15 g; pH 7.0; and a half-strength starch agar consisting of yeast extract, 1.5 g; potato starch, 5.0 g; distilled water, 1000 ml; agar, 15 g; pH 7.0. Duplicate plates of each medium were inoculated, incubated at 30°C for 5, 10, 15, and 20 days and examined at each time period directly under the microscope. Representative plates were flooded with sterile tap water, the surface growth gently scraped and samples examined microscopically for motile spores. When aerial mycelia were observed, glass cover slips (1 mm) were dropped onto the surface of the growth, inverted onto a clean glass slide and

examined under the microscope at a magnification of 1000 X.

Chemical Analysis of Whole Cells

The presence and form of diaminopimelic acid (DAP) and the presence of carbohydrates in whole-cell hydrolysates were determined by the methods of Becker et al (Appl. Microbial 12: 421-423, 1964) and Lechevalier (J. Lab. Clin. Med. 71:934-944, 1968).

Growth Characteristics

Micromonospora polytrota was cultivated on standard actinomycete media as described by Shirling and Gottlieb (Int. J. Syst. Bacteriol 16:313-340, 1966) and Waksman 1. (The Actinomycetes, Williams and Wilkins Co., Baltimore, Md. 1961, Vol. 2, pp. 328-334). Plated media were inoculated and incubated for 14 to 21 days at 35°C. The color designations assigned to the vegetative mycelium pigments consisted of a color name 2 (Descriptive Color Names Dictionary, Taylor, H.D.; Knoche, L.; Granville, W.C., Container Corp. of America, 1950) and a colorchip number 3 (Color Harmony Manual, Ed. 4, Chicago, Container Corp. of America, 1958).

Utilization of Carbohydrates

To 5 ml of the sterile, molten basal carbohydrate medium consisting of yeast extract, 5g; $CaCO_3$, 1 g; agar, 15 g; tap water, 1000 ml in 16 x 150 mm screw cap tubes, 0.5 ml of a 10% sterile solution of each test carbohydrate was added, the contents thoroughly mixed, and the tubes slanted. The slants were inoculated with Micromonospora polytrota using a sterile pipette. Tubes were observed for growth after 21 days at 35°C.

Decomposition of Organic Compounds

The decomposition of adenine, tyrosine, hypoxanthine, xanthine, xylan, urea, and allantoin were measured according to the procedures described by Lechevalier in the publication referred to herein above.

Chitin hydrolysis was measured using a modification of the procedure of Veldkamp (Veldkamp H.: "A study of the aerobic decomposition of chitin by microorganisms", Neded Landbouw Wageningen 55:127-174, 1955). Ten ml of solidified water agar dispensed into 16 x 50 mm petri dishes was overlaid with 2.5 ml of colloidal chitin agar prepared as described below. Plates were inoculated with a streak down the center, incubated

for 7, 14 and 21 days at 35°C and examined for dissolution of the chitin.

Hydrolysis of hippurate was determined using a modified Baird-Parker medium (sodium hippurate, 10 g; dextrose, 2 g; tryptone, 2 g; beef extract, 1 g; yeast extract, 2 g; $Na_2HPO_4$, 5 g; distilled water, 1000 ml; pH 7.0). The medium was dispensed in 10 ml aliquots into 25 mm tubes stoppered with Morton closures. Tubes were inoculated with the test culture, and incubated at 30°C on a rotary shaker at 250 rpm. After 7 and 14 days, the culture was tested for benzoic acid by mixing 1 ml of the culture broth, free of clumps, with 1,5 ml of 50% sulfuric acid in a 16 mm test tube. The appearance of crystals in the acid mixture after 4 hours at room temperature indicated hydrolysis of the hippurate.

Temperature Relationships

The ability of the test organisms to grow at 28°C, 35°C, 40°C and 45°C and to survive at 50°C was tested on agar slants consisting of yeast extract, 5 g; dextrose, 10 g; soluble starch, 20 g; NZ Amine A (Difco), 5 g; $CaCO_3$, 1 g; agar, 15 g; tap water, 1000 ml employing the techniques outlined by Gordon et al (Gordon RE; Barnett DA; Henderhan JE; PANGCH: Nocardia coeliaca,

*Nocardia autotrophica*, and nocardin strain. Int.J. Syst.Bacteriol. 24, 54-63, 1974).

Antibiotic Susceptibility of the Novel Strain

Test antibiotics were dissolved in the appropriate solvent at a concentration based on an activity of 1000 mcg/ml. Stock solutions were diluted in sterile distilled water to a final concentration of 500 mcg/ml and filter sterilized (0.45 mm filters, Millipore Corporation). To 10 ml of the sterile antibiotic solutions, there was added 90 ml of melted agar consisting of yeast extract, 10 g; dextrose, 10 g; agar, 15 g; tap water, 1000 ml; pH 2.0; cooled to 45°C, and 20 ml of the resulting mixture was aseptically pipetted into sterile petri dishes (100 x 150 mm). The final concentration of each antibiotic in the agar was 50 mcg/ml.

Utilization of Organic Acids

Utilization of organic acids was determined using the procedures given by Gordon *et al*, in the publication referred to above.

Morphology

Morphologically, some broth preparations of Micromonospora polytrota exhibit a vegetative mycelium formed in large clumps which are difficult to break up. The hyphae are fine, 0.8 to 1.2 microns, and are abundantly branched. The branched hyphae exhibit swelling which form unusual type structures. Spores are not formed to any substantial degree in the broth media utilized even after 10 days incubation. When present, the spores occur singly along the length of the hyphae, either attached directly to the mycelium or on sporophores.

By contrast, abundant sporulation is observed on the first and third (starch) agar media utilized in the morphological tests. Electron microscopic observation of the spore silhouettes revealed the presence of protrubances or warts along the spore surface.

Hydrolyzed whole cells contain the meso isomer of diaminopimelic acid. Arabinose and xylose are the characteristic sugars.

Macroscopic observations of the growth, or lack thereof, of M. polytrota on a variety of descriptive media are set forth on Table 1 below.

Macroscopic observations of the utilization, or lack thereof, of carbohydrates and organic acids by M. polytrota are set forth on Table 2 below.

Table 3 sets forth the growth, or lack thereof, of M.polytrota in the presence of representative antibiotics.

The table further sets forth the microorganism's growth, or lack thereof, in the presence of inorganic salts at various concentrations.

Additionally, Table 3 sets forth the microorganism's ability, or lack thereof, to hydrolyze a variety of compounds which are carbon and nitrogen sources.

TABLE 1

GROWTH CHARACTERISTICS OF M.POLYTROTA ON VARIOUS DESCRIPTIVE MEDIA

| Medium | | Growth Characteristics |
|---|---|---|
| Bennett's Agar | G: | +++, good |
| | S: | Raised, granular |
| | AM: | Present; gray bloom |
| | DFP: | Present; gray black |
| | C: | gn, charcoal |
| Czapek-Sucrose Agar | G: | +++, good |
| | S: | Raised, plicate |
| | AM: | Present, gray bloom |
| | DFP: | Present, dark gray |
| | C: | gp, black |
| Glucose-Asparagine Agar | G: | ++, moderate |
| | S: | Raised, granular |
| | AM: | Absent |
| | DFP: | Present, faint brown |
| | C: | g5po, chocolate brown |
| Glycerol-Asparagine Agar (ISP No.5) | G: | +, fair to poor |
| | S: | Granular |
| | AM: | Absent |
| | DFP: | Absent |
| | C: | g4lg, light spice brown |
| Nutrient Agar | G: | +, fair to poor |
| | S: | Granular |
| | AM: | Absent |
| | DFP: | Absent |
| | C: | g4lg, light spice brown |
| Potato Dextrose Agar | G: | ++, moderate |
| | S: | Raised, plicate |
| | AM: | Present; white to gray bloom |
| | DFP: | Absent |
| | C: | g5ml, chocolate |
| Emerson's Agar | G: | +, fair to poor |
| | S: | Flat, granular |
| | AM: | Absent |
| | DFP: | Present; gray-brown |
| | C: | g4pn, chocolate brown |

TABLE 1 (CONTINUED)

| Medium | Growth Characteristics |
|---|---|
| NZA Glucose Agar | G: +++, good |
| | S: Raised, plicate |
| | AM: Present, white to gray bloom |
| | DFP: Present; absent |
| | C: gn, charcoal |
| Yeast Extract Glucose Agar | G: +++, good |
| | S: Raised, plicate |
| | AM: Present, white to gray |
| | DFP: Present, gray |
| | C: g8pn, ebony brown |
| Tomato Paste Oatmeal Agar | G: +++, good |
| | S: Raised, powdery |
| | AM: Present; gray bloom |
| | DFP: Present; faint-gray |
| | C: gd, gray |
| Yeast Extract - Malt Extract Agar (ISP No.2) | G: +++, good |
| | S: Raised, plicate |
| | AM: Absent |
| | DFP: Present; gray |
| | C: g9pn, dark eggplant |
| Oatmeal Agar | G: +, fair to poor |
| | S: Granular |
| | AM: Present; white to gray bloom |
| | DFP: Absent |
| | C: g5po, chocolate |
| Water Agar | G: poor |
| | S: Granular |
| | AM: Absent |
| | DFP: Absent |
| | C: g4pl, dark spice brown |
| Inorganic Salts - Starch Agar (ISP No. 4) | G: ++, moderate |
| | S: Flat, granular |
| | AM: Present; gray bloom |
| | DFP: Present, gray |
| | C: g5po, chocolate brown |

TABLE 1 (CONTINUED)

| Medium | | Growth Characteristics |
|---|---|---|
| Starch Agar (Waksman No.21) | G: | +, fair to poor |
| | S: | Flat |
| | AM: | Present; white to gray bloom |
| | DFP: | Present; gray |
| | C: | g4nl, dark brown |
| Calcium Maleate Agar | G: | +, fair to poor |
| | S: | Granular, raised |
| | AM: | Absent |
| | DFP: | Absent |
| | C: | g4pg, dark luggage tan |
| Tyrosine Agar (ISP No.7) | G: | ++, moderate |
| | S: | Raised, plicate |
| | AM: | Absent |
| | DFP: | Present; gray brown |
| | C: | g8pn, ebony brown |
| Starch Agar (Gordon) | G: | +++, good |
| | S: | Raised, plicate |
| | AM: | Present; gray bloom |
| | DFP: | Present; faint brown |
| | C: | g10po, black plum |
| Casein Agar (Gordon) | G: | ++, moderate |
| | S: | Flat, granular |
| | AM: | Present; gray |
| | DFP: | Present; yellow |
| | C: | g5pi, copper brown |
| Gelatin Agar (McDade) | G: | +, fair to poor |
| | S: | Granular |
| | AM: | Absent |
| | DFP: | Absent |
| | C: | g4nl, dark brown |

G= Growth; S= Surface characteristics; AM= Aerial mycelium; DFP= Diffusable pigments; and C= Color of the growth

TABLE 2

PHYSIOLOGIC PROPERTIES OF M. POLYTROTA

| Test | M. polytrota |
|---|---|
| **Utilization of Carbohydrates:** | |
| D-Arabinose | +++, good |
| L-Arabinose | +++, good |
| Cellibiose | +++, good |
| Dulcitcl | -, poor |
| Erythritol | -, poor |
| Fructose | +++, good |
| L-Fucose | -, poor |
| Galactose | poor to fair |
| Glucose | +++, good |
| α-m-d-glucoside | -, poor |
| Glucerol | -, poor |
| Inositol | -, poor |
| Inulin | -, poor |
| Lactose | -, poor |
| Maltose | +++, good |
| Mannitol | -, poor |
| Mannose | +++, good |
| Melibiose | -, poor |
| Raffinose | -, poor |
| Rhamnose | -, poor |
| Ribose | +, fair |
| Sucrose | +++, good |
| Trehalose | +++, good |
| D-zylose | -, poor |
| **Utilization of Organic Acids:** | |
| Acetate | + |
| Benzoate | - |
| Butyrate | + |
| Citrate | + |
| Formate | + |
| Gluconate | - |
| Glucuronate | + |
| Glutamate | + |
| Lactate | + |
| Oxalate | + |
| Propionate | + |
| Pyruvate | + |
| Succinate | - |

TABLE 3

| Test | M.polytrota |
|---|---|
| **Growth in the Presence of: 50 mcg/ml** | |
| Gentamicin | + |
| Sisomicin | + |
| Neomycin | - |
| Kanamycin | + |
| Streptomycin | - |
| Erythromycin | - |
| Halomicin | - |
| Everninomicin | - |
| Rosaramicin | - |
| Cycloserine | - |
| Tetracycline | - |
| Penicillin G | - |
| Lincomycin | +++ |
| Clindamycin | +++ |
| Cephalothin | - |
| **10 mcg/ml** | |
| Rosaramicin | - |
| Chloramphenicol | - |
| Streptomycin | - |
| **Growth in the Presence of:** | |
| NaCl 1.0% | +++, good |
| NaCl 2.0% | +, fair to poor |
| NaCl 3.0% | -, no growth |
| $Na_2S_2O_3$ 1% | ++, moderate |
| 2% | -, no growth |
| 3% | -, no growth |
| 4% | -, no growth |
| **Hydrolysis of:** | |
| Adenine | - |
| Hypoxanthine | +, strong |
| Tyrosine | very weak |
| Xanthine | - |
| Xylan | - |
| Chitin | - |
| Casein | + |
| Starch | + |
| DNA | + |
| Gelatin | + |
| Hippurate | + |
| Cellulose | - |

TABLE 3 (CONTINUED)

| Test | M.polytrota |
| --- | --- |
| Breakdown of: | |
| Urea 7 d | + |
| 28 d | + |
| Allantoin | + |
| Nitrate Nitrite: | + |
| Growth at: | |
| 27°C | ++. moderate |
| 35°C | +++, good |
| 40°C | ±, poor |
| 45°C | -, no growth |
| 50°C/8 hours | +++, good |
| Litmus Milk: | No reaction |

TABLE 4

DESCRIPTION OF COLONIES OF M.ECHINOSPORA NRRL 2985, M.PURPUREA NRRL 2953, AND M.ROSARIA NRRL 3718 COMPARED TO M.POLYTROTA NRRL 12066

| Medium | M.polytrota NRRL 12066 | M.echinospora NRRL 2985 | M.purpurea NRRL 2953 | M.rosaria NRRL 3718 |
|---|---|---|---|---|
| **Bennett's Agar** | | | | |
| Growth: | good | good | good | good |
| Color: | charcoal, gn. | deep marron, g 7 1/2 pl. | terra cotta,m5pe | dark brown, g5pn |
| **Emerson's Agar** | | | | |
| Growth: | fair | good | good | fair |
| Color : | chocolate brown, g4pn. | tile red, g5n3. | burnt orange,g5nc. | dark brown,mahogany, m6pn. |
| **Tomato Paste-Oatmeal Agar** | | | | |
| Growth: | good | fair | good | fair |
| Color : | gray, gd. | dusty orange,g4lc. | burnt orange,g5n4. | dark brown,g4pn. |
| **Glucose-Asparagine Agar** | | | | |
| Growth: | moderate | poor | fair | poor |
| Color: | chocolate brown, g5po. | | bright peach,g5la. | |
| **Yeast Extract-Glucose Agar** | | | | |
| Growth: | good | good | good | good |
| Color: | ebony brown, g8pn. | maple, g4le. | deep brown,g4pl. | copper brown, g5pi. |

TABLE 4 (CONTINUED)

| Medium | M.polytrota NRRL 12066 | M.echinospora* NRRL 2985 | M.purpurea* NRRL 2953 | M.rosaria+ NRRL 3718 |
|---|---|---|---|---|
| NZA-Glucose Agar | | | | |
| Growth | good | good | good | moderate |
| Color | charcoal, gn. | burgundy, g 7 1/2 pl. | burgundy, g 7 1/2 pl. | rust tan, g5le |

TABLE 5

DISTINGUISHING PHYSIOLOGIC PROPERTIES OF MICROMONOSPORA POLYTROTA, M.ECHINOSPORA, M.PURPUREA, AND M.ROSARIA

| Test | M.sp. polytrota | M.echinospora | M.purpurea | M.rosaria |
|---|---|---|---|---|
| Carbohydrate Utilization: | | | | |
| D-Arabinose | +++, good | ±, poor | ±, poor | +++, good |
| L-Arabinose | +++, good | +++, good | +++, good | +++, good |
| Fructose | +++, good | ±, poor | ±, poor | ++, moderate |
| Galactose | ±, poor | ±, poor | ±, poor | +, fair |
| α-m-d-glucoside | -, poor | -, poor | -, poor | +, fair |
| Mannitol | -, poor | -, poor | -, poor | +++, good |
| Rhamnose | -, poor | +++, good | -, poor | +++, good |
| Ribose | +, fair | ±, poor | +, fair | +++, good |
| D-Xylose | -, poor | +++, good | +++, good | +++, good |
| Organic Acid Utilization: | | | | |
| Citrate | + | - | - | - |
| Formate | + | ± | ± | - |
| Gluconate | - | + | + | + |
| Lactate | + | + | + | - |
| Oxalate | + | - | - | - |
| Growth in the Presence of: 50 mcg/ml | | | | |
| Kanamycin | + | + | + | - |
| Erythromycin | - | - | - | + |

TABLE 5 (CONTINUED)

| Test | M.sp. polytrota | M.echinospora | M.purpurea | M.rosaria |
|---|---|---|---|---|
| Growth in the Presence of: 50 mcg/ml | | | | |
| Everninomicin | − | − | − | + |
| Rosaramicin | − | − | − | + |
| Lincomycin | + | − | − | + |
| Clindamycin | + | − | − | + |
| Growth in the Presence of: 10 mcg/ml | | | | |
| Rosaramicin | − | + | + | + |
| Chloramphenicol | − | + | + | − |
| Hydrolysis of: | | | | |
| Hypoxanthine | + | − | − | − |
| Tyrosine | ±very weak | + | + | + |
| Xylane | − | + | + | + |
| Chitin | − | + | + | + |
| Hippurate | + | − | − | − |
| Urease 7d | + | − | − | + |
| 28d | + | − | − | + |
| Allantoinase | + | − | − | − |
| Temperature: | | | | |
| 40°C | ± poor | ++, moderate | ++,moderate | ++, moderate |
| 50°C/8 hours | +++,good | ±, poor | ±, poor | +, fair |

TABLE 5 (CONTINUED)

| Test | M.sp. polytrota | M.echinospora | M.purpurea | M.rosaria |
|---|---|---|---|---|
| Sporulation Pattern: | | | | |
| Broth | Poor,7-10 days | Poor,7-10 days | Poor,no spores formed | Good, 5-7 days |
| Agar | Good, 7 days | Good, 7 days | Poor,no spores formed | Good, 7 days |
| Spore Morphology: | Slightly warty | Warts spike like 0.1 micron in length | No spores | Slightly warty |

The antibiotic complex (antibiotic AR-5) of this invention is elaborated when the M. polytrota is fermented under controlled, submerged, aerobic conditions in an aqueous nutrient medium containing assimilable sources of carbon and assimilable sources of nitrogen. Exemplary of such assimilable carbon sources are the carbohydrates. Exemplary of such assimilable sources of nitrogen are extracts from proteins. Preferred carbohydrates are tryptose, starch, dextrose, cerelose, mannitol, glucose and the like. Preferred nitrogen sources are corn steep liquor, yeast extract, soybean meal, meat peptones, casein hydrolysate and the like.

The pH of the fermentation medium and of the vegetative inoculum is adjusted prior to inoculation and is, preferably, maintained at from about 6.0 to about 8.5 by the incorporation of a buffer such as calcium carbonate in the fermentation medium or in the vegetative inoculum. The preferred pH range is from about 6.8 to about 7.2.

Production of the antibiotics of this invention may be effected at most temperatures conducive to satisfactory growth of the microorganism; e.g. between 20°C and 40°C, preferably between 27°C and

and 35°C. The most preferred temperature at which to prepare the vegetative inoculum and to conduct the fermentation is about 30°C.

In general, the nutrient media are prepared in a suitable fermentation vessel or flask, are sterilized and cooled prior to inoculation. However, the media may be stored under aseptic conditions at low temperatures prior to use.

In order to produce the antibiotic complex of this invention, a vigorously growing vegetative inoculum is preferred. In general, the inoculum preparation is carried out in two or more stages. For large scale fermentations (e.g. about 50 liters), it is preferred that the inoculum be prepared in three stages.

Suitable media for preparing vegetative inocula are as follows:

| Medium A | | Medium B | |
|---|---|---|---|
| Beef Extract | 3 g | Potato Dextrin PD650 | 50 g |
| Tryptose | 5 g | Soy Grits | 35 g |
| Dextrose | 1 g | Dextrose | 5 g |
| Potato Starch | 24 g | Calcium Carbonate | 7 g |
| Yeast Extract | 5 g | Cobalt Chloride | 238 g |
| Calcium Carbonate | 2 g | Tap Water to | 1.C liter |
| Tap Water to 1.0 liter | | | |

Two ml of freshly thawed whole broth of M. polytrota is used to inoculate 50 ml of sterile medium A. The flask is incubated at about 30°C for from about 48 to about 96, preferably about 72 hours on a gyratory shaker at about 300 rpm and a 2 inch throw. Twenty-five ml of the first inoculum is used to inoculate a series of 500 ml portions of sterile Medium B in two liter Erlenmeyer flasks. The flasks are incubated with gyratory agitation at about 30°C for from about 24 to about 72, preferably 48 hours. Twenty-five ml of the second inoculum is used to inoculate each of a series of 500 ml portions of sterile Medium B in two liter Erlenmeyer flasks. The flasks are incubated with gyratory agitation at about 30°C for from about 24 to about 72, preferably 48 hours.

The following medium has been found to provide both satisfactory and reproducible yields of antibiotic production

Medium C

| | |
|---|---|
| Staley J Starch | 50 g |
| Distillers Solubles | 7.5 g |
| Pharamedia | 5.0 g |
| Cerelose | 5.0 g |
| Tap water to | 1.0 liter |

For the production of the antibiotic complex ten liters of sterile Medium C are inoculated with 500 ml of the second stage inoculum prepared as described above. The fermentation mixture is incubated at from about 27° to about 35°C, preferably at about 30°C with rotary agitation and aeration at about 350 rpm and at 0.35 vvm, respectively. The pH of the fermentation mixture is maintained at from about 6.8 to about 7.2 by the addition of dilute alkali.

The fermentation is permitted to proceed for from about 48 to about 84, preferably about 72 hours before commencing to monitor for antibioti activity. To monitor the fermentation, a sample of the whole broth is withdrawn and extracted with a water immiscible organic solvent. Exemplary of such solvents are methylene chloride, chloroform, benzene, toluene, ethyl acetate, amyl acetate or the like. The preferred solvent is ethyl acetate. The monitoring is conducted by thin layer chromatography on silica gel using the lower phase of a solvent system consisting of chloroform: methanol:petroleum ether: water in the ratio by volume of 3:3:1:1. Quantitation of antibiotic production may be made using High Performance Liquid Chromatography (HPLC) or other analytical techniques known in the art.

When peak antibiotic concentration is attained, the antibiotic AR-5 complex may be isolated by extraction as described in the monitoring procedure using about two volumes of immiscible organic solvent per extract and by extracting the broth twice. The extracts are combined, evaporated to a residue and dissolved in methanol. Precipitation of the product by the addition of petroleum ether to the methanol solution yields the antibiotic AR-5 complex.

Separation of the antibiotic complex into its components can be effected by chromatography on Sephadex LH-20 using a concentrate of an ethyl acetate extract. Elution of the bioactive material can be effected with ethanol. The ethanol eluate containing the bioactive fractions is concentrated and absorbed onto a silica gel column. Elution is effected using the previously described chlorofrom, methanol, petroleum ether and water system. The column is monitored by the use of thin layer chromatography using silica gel plates and the solvent system being used to elute the column. Fractions having $R_f$ of 0.51 and 0.43 contain antibiotic AR5-1, antibiotic AR5-2, and the corresponding 12, 13-desepoxy-12,13-dehydro derivatives thereof, designated 12,13-desepoxy-12,13-dehydro antibiotic AR5-1 and 12,13-desepoxy-12,13-dehydro antibiotic AR5-2.

Co-produced in the above described fermentation is gentamicin C complex, a well known antibiotic (U.S. Patents 3,091,572 and 3,136,704), which consists of three distinct entities, namely gentamicin $C_1$, gentamicin $C_{1a}$ and gentamicin $C_2$. The above described solvent extraction procedure (by which the components of antibiotic AR-5 complex are isolated) leaves the gentamicin complex in the broth. It can be isolated from the fermentation broth by known techniques such as the following isolation steps: acidification of the broth to pH 2.0 (e.g. with sulfuric acid), filtration, neutralization of the filtrate with ammonium hydroxide, absorbtion to an IRC-50 resin column in the ammonium cycle, desorbtion of the gentamicin components with dilute ammonium hydroxide, concentration of the eluate and lyophilization.

Antibiotic AR5-1, antibiotic AR5-2 and the other compounds of formula I, wherein A together with the carbon atoms of positions 12 and 13 represents an oxirane ring, can be converted to their respective 12,13-desepoxy-12,13-dehydro derivative by reduction. This process can be carried out with alkali metal bromides or preferably with alkali metal iodides in an organic acid at above ambient temperature. The

carboxylic acids preferably contain 1 to 4 carbon atoms. This reaction can also be carried out in aromatic hydrocarbons, such as for example benzene, toluene or xylene, containing concentrated hydroiodic acid. Another possibility resides in treating the starting material with phosphorus trialkoxides, trialkyl phosphines, triphenyl phosphines or hexa-alkyl phosphoimidates.

A medium for effecting this reaction is ——————— potassium iodide in refluxing acetic acid. The reaction product may be advantageously isolated by diluting the reaction mixture with ice-water, extracting the product with a water immiscible organic solvent, washing the extract and isolating the product therefrom. The product of this process consists of substantial amounts of both the cis and trans isomers of the reduction product.

This reduction process can also be performed in a preferably dilute mineral acid solution containing chromous ions in an inert atmosphere. The reducing agent, i.e. the chromous ions, are advantageously supplied in the form of a solution containing a chromous salt wherein the anion is derived from a mineral acid, e.g. chromous chloride, chromous

sulfate or chromous iodide. The preferred reducing agent is chromous chloride which may advantageously be prepared by the procedure described in Inorganic Synthesis, Volume III, pages 148-150, published by McGraw-Hill (1950) ——————————————————. It is preferred that the chromous chloride solution be freshly prepared immediately before use to mitigate against chromic ion formation. The product is rich in the trans isomer and, therefore, does not require chromatographic separation of the isomers.

10,11-Dihydro antibiotic AR5-1 (a compound of formula I wherein M is oxo, R is hydrogen, Z is the group of formula II wherein X is hydroxy and Y is hydrogen, A together with the carbon atoms of positions 12 and 13 is an oxirane ring and B is a single bond between the carbon atoms of positions 10 and 11) is the metabolite of antibiotic AR5-1. It can be obtained by enzymatic, preferably metabolic, reduction of antibiotic AR5-1. Antibiotic AR5-1 can, for example, be fed to animals (e.g. dogs) capable of reducing it at the double bond between the carbon atoms of positions 10 and 11, and the 10,11-dihydro antibiotic AR5-1 is isolated from the urine of these animals by extraction. The extraction is preferably carried out with water-immiscible solvents, such as for example ethylacetate.

The compounds of formula I, derived from the antibiotic complex AR-5 can be prepared by an appropriate process or a combination of processes described below. The processes are known in the art.

Compounds of the general formula I, wherein R is hydrogen or hydroxy, M is oxo, ———— A and B represent double bonds between the carbon atoms of positions 12 and 13, and 10 and 11 respectively, and Z is $-CH_2OH$, can be prepared by hydrolysis of compounds of formula I, wherein R, M, A and B are defined as above and Z is $-CH_2O$-glycosyl. This hydrolysis can be carried out by acid hydrolysis. Usually this hydrolysis is carried out at elevated temperatures (e.g. 60-100°C, preferably at about 100°C), using dilute acid, preferably an inorganic acid (e.g. 0.5 $NH_2SO_4$). The desired product is isolated by adjusting the solution to pH 9.0, followed by extraction, and known cleaning steps.

When for example, this process is used for the hydrolysis of 12,13-desepoxy-12,13-dehydro antibiotic AR5-1 and/or 12,13-desepoxy-12,13-dehydro antibiotic AR5-2, the reaction can be carried out with 0.5 N sulfuric acid at about 96°C for 5 hours.

A preferred method for the preparation of the desmycinosyl compounds (compounds of formula I wherein Z is

$-CH_2OH$) is the hydrolysis of the respective compound of formula I wherein Z is $-CH_2O$-glycosyl of formula II, wherein Y in combination with X is oxo, by means of methanolic silica gel. For example 4"-dehydro-12,13-desepoxy-12,13-dehydro antibiotic AR5-1 (preferably the 2'-ester, for example 2'-acetate thereof) is treated with methanolic silica gel at room temperature during 4 days. The desired desmycinosyl-12,13-desepoxy-12,13-dehydro antibiotic AR5-1 is isolated from the reation mixture, e.g. by column chromatography, and is obtained in high yields.

The desmycinosyl compounds of the general formula III

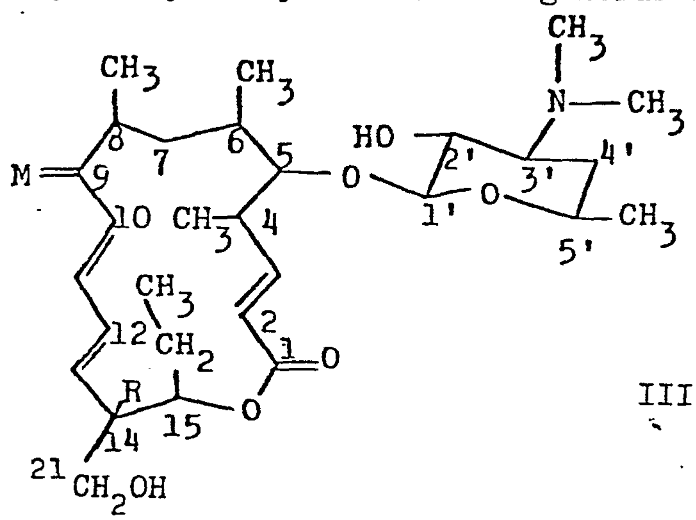

III

can be converted to the corresponding compounds of formula I wherein Z is $-CHO$, $-CH_2NH_2$, $-CH_2NHR^2$, $-CH_2NR^2R^3$, $-CH_2OR^2$, $-CH_2OCOR^2$, $-CH_2NHCOR^2$, $-CH_2NR^3COR^2$,

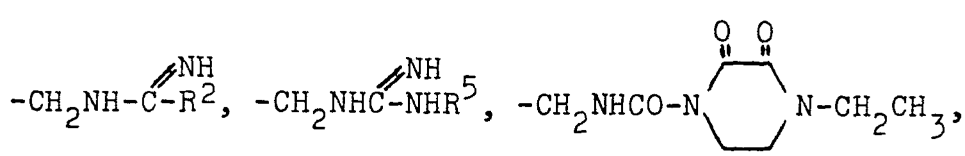

$-CH_2O$-glycosyl excluding $-CH_2O$-mycinosyl and $-CH_2O$-

(3"-desmethyl-mycinosyl), $-CH_2$-ureido or $-CH_2$-thioureido, wherein $R^2$ and $R^3$ independent of each other represent an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen, or in the amino groups one of substituents $R^2$ and $R^3$ is hydrogen and the other is the group $-(CH_2)_mOR^4$ or - 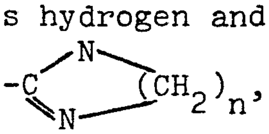, wherein m is an integer of from two to ten, n is an integer of from 2 to 6 and $R^4$ is an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen; and $R^5$ is hydrogen, $R^2$ or $-CH_2CH_2$- 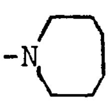.

The compounds of formula III can be oxidized to the corresponding compounds of formula I wherein Z is -CHO and M is oxo. This oxidation can, for example, be carried out by means of the Jones reagent (chromic anhydride in dilute sulfuric acid).

The compounds of formula III can be subjected to glycoside formation. This glycoside formation is

carried out according to known methods, such as reaction of a compound of formula III or a reactive derivative thereof with the appropriate sugar in the presence of a catalyst (e.g.1% HCl) and in the absence of water.

In particular, the glycosides of this invention may be prepared by the processes set forth in Angew.Chem.International Edition 1974, No.3, pages 157-170 and the references cited therein. It is preferred to use compounds of formula III wherein M is oxo. Examples of sugars are β-D-glucose, β-D-allose, 2-deoxy-β-D-glucose, 2-deoxy-β-D-allose, 2,3-di-O-methyl-β-D-glucose, 2,3-di-O-methyl-β-D-allose, 2-deoxy-3-O-methyl-β-D-allose, 2-O-methyl-β-D-allose, 6-deoxy-β-D-allose, 6-deoxy-2,3-di-O-methyl-β-D-glucose, 2,6-dideoxy-3-O-methyl-β-D-allose, 6-deoxy-2-O-methyl-β-D-allose, β-D-galactose, 2,3-di-O-methyl-β-D-galactose, 2-O-methyl-β-D-galactose, 2-O-methyl-β-D-galactose, 6-deoxy-2,3-di-O-methyl-β-D-galactose, 6-deoxy-2-O-methyl-β-D-galactose, 2-O-methyl-β-D-galactose, 6-deoxy-β-D-galactose and 6-deoxy-2,3-di-O-methyl-β-D-galactose.

The compounds of formula III can also be etherified in position 21 to form corresponding compounds of formula I wherein Z is $-CH_2OR^2$, $R^2$ being defined as above. The etherification is carried out according to known methods. Hydroxy groups of other positions

and amino groups of the starting material have to be protected by usual protecting groups during the etherification process. (A convenient way of protecting hydroxy groups is to acylate the starting material in the relevant position, especially the 2'-hydroxy group, and to subject the product of the etherification process to solvolysis, whereby the free hydroxy group(s) in positions other than position 21 is (are) regenerated). The etherification can be carried out by reacting the compound of general formula III with the respective $R^2$-iodide and silver oxide or alternatively with sodium-hydride and then with the respective $R^2$-halide in an organic solvent such as tetrahydrofuran.

The compounds of formula I, wherein Z is -CHO can be transformed to those compounds of formula I, wherein Z is $-CH_2NH_2$, $-CH_2NHR^2$ or $-CH_2NR^2R^3$ ($R^2$ and $R^3$ are defined as above) by reductive amination. For example, this transformation can be carried out with a reducing agent such as sodium cyanoborohydride and an aminating agent such as ammonium chloride to yield the C-21 primary amine derivative. The substituted amino derivatives (Z being $-CH_2NHR^2$ or $-CH_2NR^2R^3$ ) can be obtained if in this reaction an $R^2$- or $R^2$-$R^3$ - substituted aminating agent is used.

If the starting material used in this process is a compound of formula I wherein M is oxo, this oxo-group is reduced to the 9($\alpha$,$\beta$)-dihydro group (M being H, OH). If desired this so obtained product can be converted to the corresponding 9-oxo compound by known oxidation procedures such as procedures using manganese dioxide or the Jones' reagent.

Compounds of formula I wherein Z is $-CH_2NH-C(=NH)-R^2$, $-CH_2NHC(=NH)-NHR^5$,

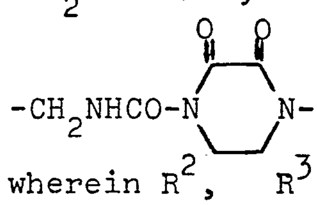

$-CH_2CH_3$, $-CH_2$-ureido or $-CH_2$—thioureido, wherein $R^2$, $R^3$ and $R^5$ are defined as above can be prepared by reacting a compound of formula IV,

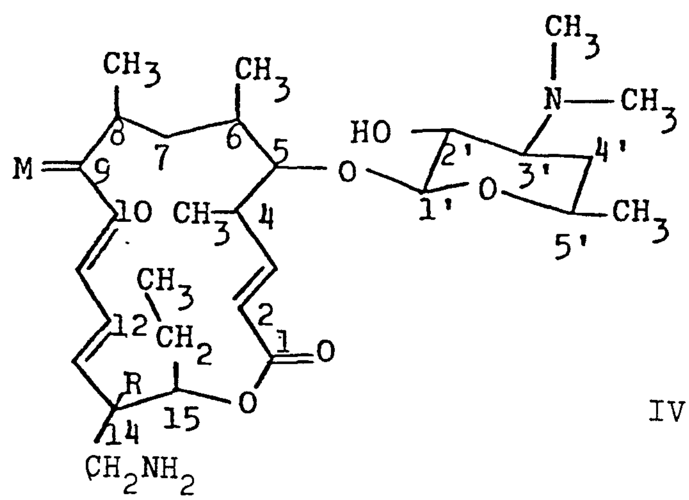

IV

wherein M and R are defined as above, or a 2'-ester thereof with a compound of formula (V) L-Q, wherein Q is the moiety to be introduced in the primary amino

group at position 21 of the starting material and L is a leaving group. Suitable leaving groups are groups such as for example halo, alkoxy and thioalkyl.

A compound of formula I, wherein Z is $-CH_2NHR^2$ or $-CH_2NR^2R^3$ can be prepared by reacting the corresponding compound of formula IV with an aldehyde ($R^2CHO$) or a ketone ($R^2COR^3$), respectively, in the presence of a reducing agent such as for example sodium cyanoborohydride and sodium borohydride.

The reaction is carried out in a non-reactive solvent such as a lower alcohol (e.g. methanol). If in the starting material of formula IV substituent M is oxo, this 9-oxo group is reduced by this reaction as well and a mixture of the 9-α- and 9-β-dihydroderivative is obtained. This mixture can be resolved by known methods and, or if desired the 9-dihydro derivative can be oxidized to form the corresponding compound of formula I wherein M is oxo.

The compounds of formula I, wherein Z is $-CH_2NHCOR^2$ or $-CH_2NR^3COR^2$ can be prepared by acylating the appropriate compound of formula IV or a corresponding compound containing a secondary aminogroup ($-NHR^2$) at the carbon atom of position 21. A suitable acylating agent is the anhydride or the halogenide of the acid $R^2COOH$, wherein $R^2$ is as defined above.

A compound of formula I (defined above), wherein M is oxo can be prepared from the corresponding 9-hydroxy compound by oxidation. Suitable agents are for example manganese dioxide and Jones' reagent (chromic anhydride in dilute sulfuric acid is added to a solution of the starting material in a non reactive solvent, e.g. acetone).

If in the starting material Z is $-CH_2OH$ or X in the group of formula II is OH, this group is oxidized as well.

A compound of formula I wherein M is (h,OH) and A, B, R and Z are as defined above, with the proviso that Z is other than -CHO, and that, if Z is the group of formula II Y is hydrogen, can be prepared by reduction of a compound of formula I defined as above, wherein M is O. Suitable reduction procedures use sodium borohydride in a non-reactive solvent such as an alcohol (e.g. methanol), sodium cyanoborohydride in aqueous acidic buffer (pH3-5) or lithium tri-tertiary butoxy aluminium hydride ($LiAlH[OC(CH_3)_3]_3$, preferably in a non-reactive solvent such as for example tetrahydrofurane. By these procedures a mixture of respective 9-α-dihydro derivative and the 9-β-dihydro derivative is obtained. The components of these mixtures can be isolated by methods known in the art such as for example column chromatography.

By this reaction an oxo group in position 4" and 21 in the starting material is reduced as well and the corresponding 4"-(H,OH)- derivative and/or 21-$CH_2OH$ derivative is obtained.

In particular, by subjecting antibiotic AR5-1, antibiotic AR5-2, 10,11-dihydro-11-amino antibiotic AR5-1, 10,11-dihydro-11-amino antibiotic AR5-2, 4"-dehydro antibiotic AR5-1, 4"-dehydro antibiotic AR5-2 or esters of these compounds to this reduction procedure, the corresponding derivatives are obtained containing the group (H,OH) in position 9 and 4".

Compounds of the general formula VIII or IX

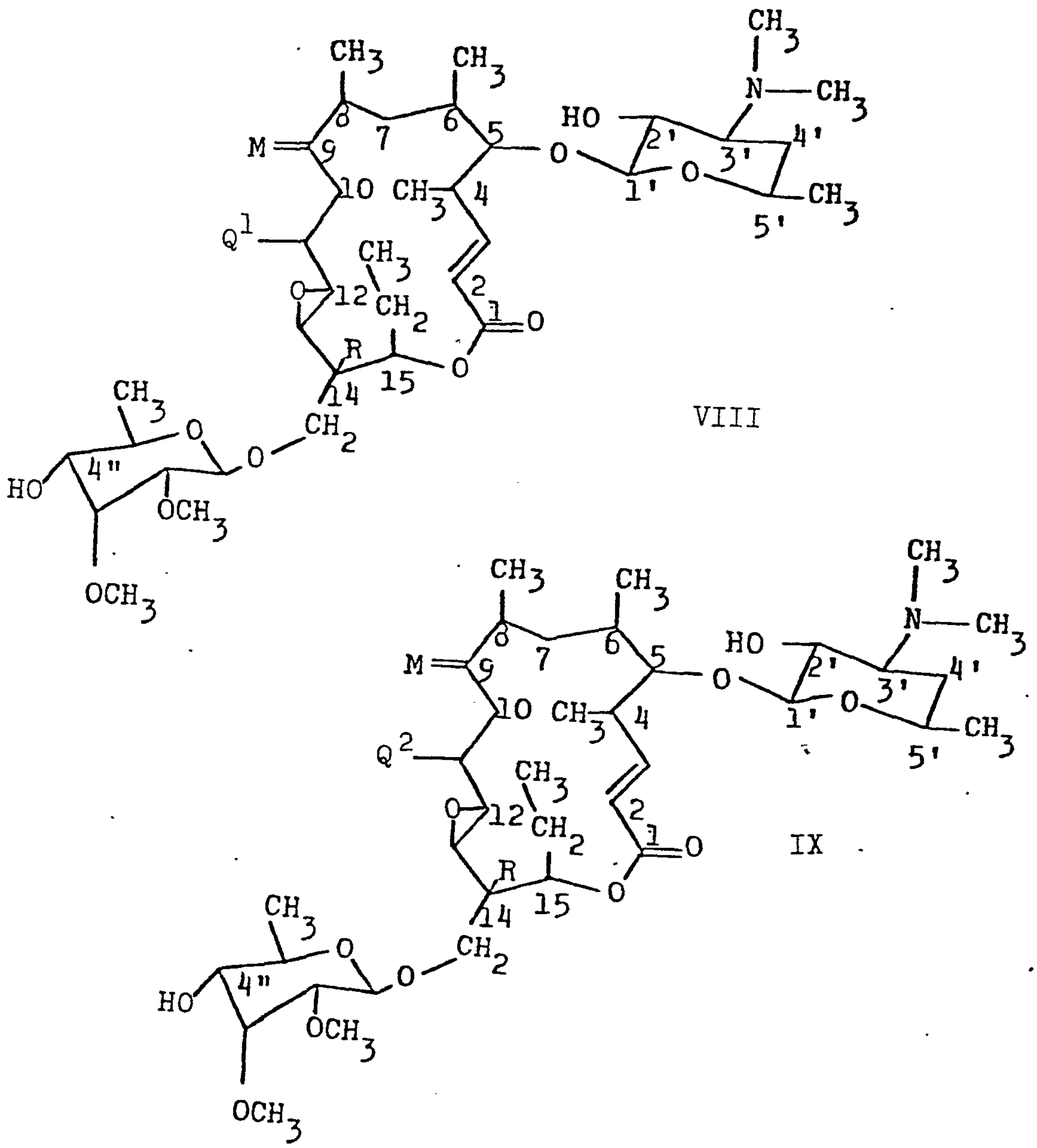

VIII

IX

wherein R is H or OH, M is O ; $Q^1$ is $-NH_2$, $-NHR^6$, $-NR^6R^7$, $-NHC(NH)NH_2$, $-NHC(NH)NHR^6$, an amino acid residue, an esterified amino acid residue or a heterocyclic radical of 3 to 7 members containing either nitrogen as the only heteroatom or nitrogen and a second heteroatom which is nitrogen, oxygen, or sulfur; and $-Q^2$ is $-S-R^6$, thioxanthyl, $-S-R^{10}-NHR^6$, $-S-R^8$, $S-R^9$, $-S-R^{10}-R^9$, $-S-R^{10}-R^8-R^9$, $-S-R^8-R^9$, $-S-R^{10}-C(O)OR^6$, $-S-R^{10}-COOH$, a sulfur containing amino acid residue, or a sulfur containing esterified amino acid residue; and $R^6$, $R^7$, $R^8$, $R^9$ and $R^{10}$ are as defined for formula I above, can be prepared by reacting antibiotic AR5-1, antibiotic AR5-2 or ——————————————— the esters of these compounds with an appropriate compound of the formula $HQ^1$ (VI) or $HQ^2$ (VII) respectively, or with a reactive derivative of the compound of formula VI or VII. In general it is preferred to use antibiotic AR5-1 or antibiotic AR5-2 as starting material.

This process is discussed in more detail for the preparation of selected compounds or selected groups of compounds. However, it is understood that the features of the process described can be applied to other compounds of formula VIII and IX as well.

Compounds of formula VIII, wherein M is oxo and

$Q^1$ is $-NH_2$, $-NHR^6$ or $-NR^6R^7$ can be prepared by reacting antibiotic AR5-1 or antibiotic AR5-2 with the appropriate compound of formula VI, $HQ^1$($NH_3$, $NH_2R^6$ and $NHR^6R^7$), or a reactive derivative thereof in a non-reactive solvent. Suitable solvents are for example low molecular weight alcohols (preferably methanol).

The so obtained 11-$Q^1$-derivative can be isolated by removal of the solvent and can be purified by methods generally known in the art such as chromatography or salt formation and crystallization, followed by regeneration of the free amine.

A compound of formula VIII, wherein M is oxo and $Q^1$ is -NH-C(NH)$NHR^6$ can be prepared analogously by reacting antibiotic AR5-1 or antibiotic AR5-2 with $R^6$-guanidine ($NH_2C(NH)NHR^6$).

A compound of formula VIII, wherein M is oxo and $Q^1$ is an amino acid or esterified amino acid residue can be prepared by reaction of antibiotic AR5-1 or antibiotic AR5-2 with the appropriate amino acid or its ester for example of the general formula X

H

|

$NH_2$-C-$CO_2R^7$

|

$R^6$

or its salt, e.g. its hydrochloride, in a non-reactive solvent such as for example a cyclic ether (e.g. tetrahydrofuran) or a dialkylacylamide (e.g. dimethylformamide), in the presence of a tertiary base such as triethylamine. If the amino acid is used, it is preferred to use its salt in a buffered solution (pH7). The reaction is usually carried out in the presence of a slight excess of a tertiary base such as triethylamine.

Compounds of formula VIII, wherein $Q^1$ is a heterocyclic radical as defined above, especially compounds wherein M is oxo and $Q^1$ is thiomorpholino, 1-methyl-4-piperazinyl, morpholino, or 1-piperidinyl can be prepared by reacting antibiotic AR5-1 or antibiotic AR5-2 with the appropriate compound of formula VI, or its reactive derivative, wherein $Q^1$ is defined as above. The raction is carried out using the amine as the solvent.

A compound of formula IX, wherein M is oxo and $Q^2$ is as defined above, preferably $-SR^6$, $-S-R^{10}-NH-R^6$ (especially $-S-(CH_2)_2NH-R^6$), $-S-CH_2-$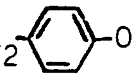$-O-CH_3$, $-S-C(S)-SR^6$, $-S-R^{10}-C(O)OR^6$, $-S-R^{10}-COOH$ (preferably those wherein $R^{10}$ is $-(CH_2)_n-$, wherein n is an integer from 2 to 6), $-S-C(CH_3)_2-CH(NH_2)-COOH$ or $-S-CH_2-CH(NH_2)COOH$ can be prepared by reacting antibiotic AR5-1 or antibiotic AR5-2 with the appropriate compound of formula VII, wherein $Q^2$ is defined as above, or its reactive derivative. Preferably the reaction is carried out in a non-reactive solvent such as for example an aqueous mixture of a lower alcohol (e.g.methanol),a cyclic ether (e.g.tetrahydrofuran) and a dialkylacylamide (e.g.dimethylformamide. This process can for example be used for the preparation of compounds of formula IX, wherein $Q^2$ is derived from a sulfur containing amino acid such as cysteine, homocysteine, pinicillamine and glutathione or from a mercaptane, such as an alkyl, aryl or aralkyl mercaptan such as an ethyl, butyl, phenethyl, or p-methoxy benzyl mercaptan.

A compound of formula VIII, wherein $Q^1$ is $-NH-C(O)NHR^6$ or $-NHC(S)NHR^6$, R is H or OH and M is O or (H,OH) can be prepared by reacting a corresponding compound of formula VIII, wherein $Q^1$ is amino with an isocyanate or isothiocyanate of the general formula $R^6NCO$ (XI) or $R^6NCS$ (XII) respectively, usually in a non-reactive organic solvent such as behzene or toluene.
Preferably substituent M in the starting material is oxo.

A compound of formula VIII, wherein $Q^1$ is $-NHR^6$, $-NR^6R^7$ or an esterified amino acid residue $-NH\overset{R^6}{\overset{|}{C}}H-COOR^7$, R is H or OH and M is O or (H,OH), can be prepared by reacting as corresponding compound of formula VIII, wherein $Q^1$ is $-NH_2$ with the appropriate aldehyde ($R^6CHO$), ketone ($R^6COR^7$) or 2-oxo-carboxylic acid ($R^6C(O)COOR^7$) in the presence of a reducing agent such as sodiumcyanoborohydride, sodium borohydride or lithium tri-tertiary butoxy aluminium hydride. The reaction is carried out in a non-reactive solvent such as a lower alcohol (e.g. methanol), or tetrahydrofuran. If in the starting material of formula VIII substituent M is oxo, this 9-oxo group is reduced by this reaction as well and a

mixture of the 9-α-and 9-β-dihydro derivative is obtained. This mixture can be resolved by known methods and, if desired the 9-dihydro derivative can be oxidized to form the corresponding compound of formula VIII wherein M is oxo and $Q^1$ is as defined above in this paragraph. It is preferred to use 2'-esters in this procedure.

A compound of formula VIII, wherein $Q^1$ is $-NHC(O)R^6$, $-NHC(O)OR^6$, -NHC(O)- 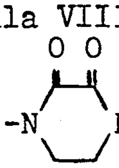 $-CH_2CH_3$, or $-N{=}CHR^6$, R is H or OH and M is O or (H,OH), can be prepared by reacting a corresponding compound of formula VIII, wherein $Q^1$ is $-NH_2$ with a compound containing, besides a leaving group, the group $-C(O)R^6$, $-C(O)OR^6$, -C(O)- 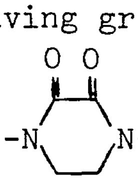 $CH_2CH_3$ or $=CHR^6$, respectively. The reaction is carried out in a non-hydroxylic solvent such as tetrahydrofuran. It is preferred to use the 2'-ester of the starting material mentioned above and to apply this process to a compound wherein M is oxo.

Compounds of the general formula XIII,

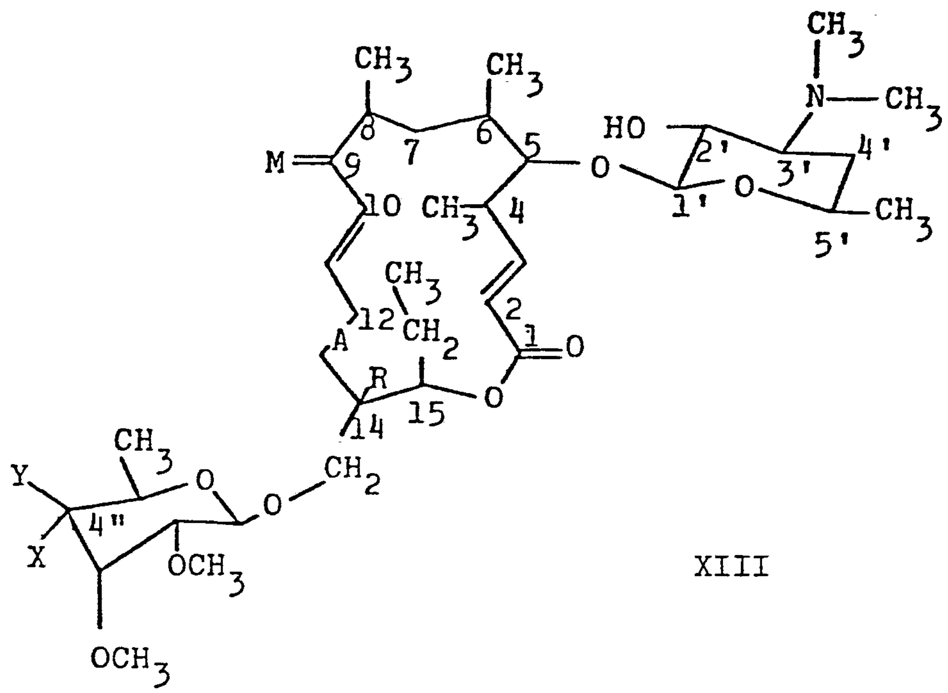

XIII

wherein R is H or OH; M is oxo or (H,OH); A represents a double bond between the carbon atoms of positions 12 and 13 or, together with the carbon atoms of positions 12 and 13, an oxirane ring; and X is hydrogen, OH, mesyloxy, tosyloxy, trifluoromethanesulfonyloxy, halo, azido, amino, isocyanato, isothiocyanato, guanidino, $-NHR^6$, $-NR^6R^7$, $-NHR^8$, $-NHC(O)OR^6$, $-NHC(O)OR^8$, $-NHC(NH)R^6$, $-NH(CH_2)pOH$ or $-NH(CH_2)pOR^{10}$, wherein p is an integer of from 2 to 18, or $-NHCOR^6$ when Y is hydrogen; or Y in combination with X is oxo, $=N-NH_2$, $=N-NHR^{11}$ or $=NR^{11}$; wherein $R^6$ and $R^7$ independent of each other represent an alkyl group containing 1 to 18 carbon atoms, or an aralkylgroup containing 7 to 18 carbon atoms; $R^8$ is an aryl group; $R^{10}$ is an alkyl group containing 1 to 18 carbon atoms

and $R^{11}$ is an alkyl group containing 1 to 18 carbon atoms, an aralkyl group containing 7 to 18 carbon atoms or a heterocyclic group containing 3 to 7 members wherein the hetero atom(s) is (are) selected from sulfur, nitrogen and oxygen; can be prepared by subjecting a compound of the general formula XIV

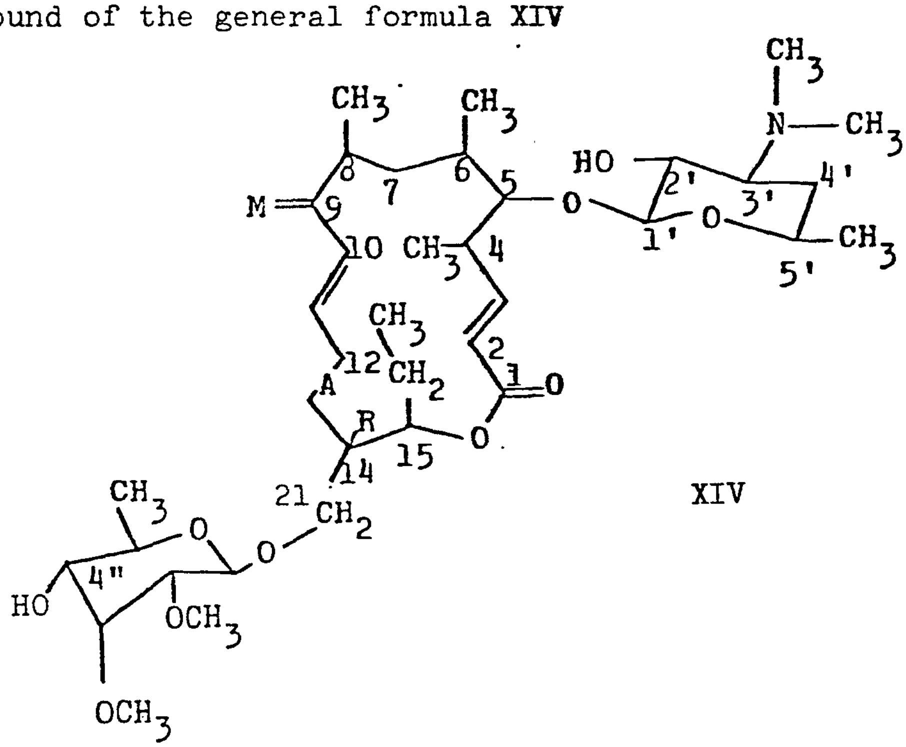

XIV

to one or more of the process steps described further below. In formula XIV,R is H or OH; M is oxo or (H,OH); A represents a double bond between the carbon atoms of positions 12 and 13 or, together with the carbon atoms of positions 12 and 13, an oxirane ring. It is preferred to use the starting compound in the form of its 2'-ester and, if M is (H,OH) its 9,2'-diester. In the starting material (XIV) of these process steps substituent R and M and A is identical to R, M and A of the desired end product. (In exceptional cases

however, it can be advantageous to use a starting material of formula XIV, wherein M is different from M in the desired end product and to subsequently introduce the desired substituent M.) The process steps are very useful for preparing compounds of the general formula XIII wherein M is oxo. Inversion occurs in several of these process steps. The epimere of a compound of formula XIII can be prepared by carrying out one or more of the process steps in appropriate order of sequence.

The process steps per se are known in the art. It is preferred to use the starting materials in the form of their esters, especially the 2'-esters. Esters derived from carboxylic acids containing 2 to 5 carbon atoms, preferably derived from acetic acid, are useful. In the following description the esterification of the starting material and the solvolysis of the end product (if the end product shall be in the free form) is not specifically mentioned.

For the preparation of a compound of formula XIII, wherein X is mesyloxy the ester of an appropriately substituted compound of formula XIV is reacted with mesylchloride in dry pyridine at room temperature, (e.g. the 2'-ester of antibiotic AR5-1 or AR5-2

is subjected to this reaction to obtain its mesylate.) Tosylates and trifluoromethene sulfonates (triflates) of the compounds of formula XIII can be prepared accordingly.

For the preparation of a compound of formula XIII, wherein X together with Y represents oxo the ester of an appropriately substituted compound of formula XIV is subjected to oxidation. The oxidation is carried out as described by Pfitzner and Moffatt, J.A.C.S. 1963, 85, 3027 ff.

For example this process can be applied to 2'-acetyl-antibiotic AR5-1 or 2'-acetyl antibiotic AR5-2 to obtain 2'-acetyl-4"-dehydro antibiotic AR5-1 or 2'-acetyl-4"-dehydro antibiotic AR5-2, respectively.

For the preparation of a compound of formula XIII, wherein X is azido, the ester of the

corresponding compound of formula XIII, wherein X is trifluoromethane sulfonyloxy, is reacted with sodium azide. In this reaction inversion occurs. For example a 2'-ester of antibiotic AR5-1 or AR5-2 can be subjected to this reaction to obtain the corresponding 4"-epi-azide.

For the preparation of a compound of formula XIII, wherein X is halo, the ester of the corresponding compound of formula XIII, wherein X is trifluoromethane sulfonyloxy, is reacted with an alkali metal halide in a non reactive solvent such as acetone. In this reaction inversion occurs. If, for example, this process step is applied to the 2'-ester of the triflate of antibiotic AR5-1 or antibiotic AR5-2 (obtained above), using sodium iodide in acetone, the corresponding 4"-epi-iodide is obtained (wherein iodo at position 4" has the opposite configuration to that of the hydroxy group of antibiotic AR5-1 or antibiotic AR5-2 obtained by fermentation).

For the preparation of a compound of formula XIII wherein X is hydroxy which has the opposite configuration to that of the hydroxy group of antibiotic AR5-1 or antibiotic AR5-2 obtained by fermentation, the corresponding triflate as

obtained above (obtained without inversion) is reacted with sodium acetate in acetic acid (see e.g. JACS 83, 3235, 3244 (1961). The so obtained 4"-acetate is epimeric to the triflate and upon hydrolysis under basic conditions yields the 4"-epi-hydroxy derivative. By this hydrolysis also the other ester group(s) in position 2' and possibly 9 are hydrolized. By this process the epimere of antibiotic AR5-1 and AR5-2 can for example be obtained.

For the preparation of a compound of formula XIII, wherein X is O=C=N- or S=C=N-, the ester of a corresponding compound of formula XIII, wherein X is azido, is reacted with triphenylphosphine and carbon dioxide or triphenylphosphine and carbon disulfide, respectively. For example this process can be applied to the 2'-ester of the 4"-epi-azide of antibiotic AR5-1 or antibiotic AR5-2 obtained as described above, to provide the corresponding isocyanate or isothiocyanate.

The ester of the 4"-epi-azide (compounds of formula XIII wherein X is azido)obtained as described above can be converted to the corresponding 4"-amino compound by reaction with triphenylphosphine according to Chem.Ber. 89, 2433 (1956). In this process inversion does not occur so that the 4"-epi-amine derivative of the compound of formula XIV or its ester is obtained.

For example, the 2'-ester of 4"-deoxy—4"-epi-azido antibiotic AR5-1 or AR5-2 obtained according to the above described process is subjected to this process followed by hydrolysis to obtain 4"-deoxy-4"-epi-amino antibiotic AR5-1 or AR5-2.

The ester of the 4"-epi-halo compound of formula XIII obtained above can be converted to the corresponding 4"-amino compound by reaction with hexamethylene tetramine. In this reaction inversion occurs and the 4"-deoxy—4"-amino derivative (or its ester) of the compound of formula XIV is obtained. If for example the ester of 4"-deoxy-4"-epi-iodo-antibiotic AR5-1 or AR5-2 obtained above is subjected to this reaction 4"-deoxy-4"-amino antibiotic AR5-1 or 4"- deoxy -4"-amino antibiotic AR5-2, respectively, is obtained.

The ester of the 4"-epi-halo compound of formula XIII obtained above can be dehalogenated to the corresponding 4"-deoxy derivative. The dehalo-

genation can be carried out by reaction with tributyl tin hydride, preferably in toluene. If for example the ester of 4"-deoxy-4"-epi-iodo antibiotic AR5-1 or -AR5-2 (obtained according to the process described above) is subjected to this reaction and subsequently subjected to solvolysis, obtained is 4"-deoxy antibiotic AR5-1 or 4"-deoxy antibiotic AR5-2, respectively.

A compound of formula XIII, wherein X is $-NHR^6$, $-NR^6R^7$ or $-NHR^8$ can be prepared by reacting the corresponding 4"-amino compound (obtained according to either one of the above described processes), preferably its ester, with an aldehyde ($R^6CHO$ or $R^8CHO$) or ketone ($R^6COR^7$), respectively, in the presence of a reducing agent such as sodium cyanoborohydride, sodium borohydride or lithium tri-tertiary butoxy aluminium hydride. The reaction is carried out in a non-reactive solvent such as a lower alcohol (e.g.methanol). $R^6$, $R^7$ and $R^8$ are as defined above.

If in the starting material of formula XIII substituent M is oxo, this 9-oxo group is reduced by this reaction as well and a mixture of the 9-α- and

9-β-dihydroderivative is obtained. This mixture can be resolved by known methods and, if desired the 9-dihydro derivative can be oxidized to form the corresponding compound of formula XIII wherein M is oxo.

A compound of formula XIII, wherein X is $-NHC(O)OR^6$ or $-NHC(O)OR^8$ can be prepared by reacting the corresponding 4"-amino compound (obtained by either one of the above described processes), preferably in the form of its ester, with the appropriate chloroformate ($ClCOOR^6$ or $ClCOOR^8$), e.g. methyl, ethyl, benzyl or phenyl chloroformate, in the presence of sodium carbonate and water. $R^6$ and $R^8$ are as defined above.

A compound of formula XIII, wherein X is $-NHCOR^6$ ($R^6$ being as defined above) can be prepared by reacting the corresponding 4"-amino compound obtained according to either one of the above described processes, preferably in the form of its ester with the anhydride or halide, preferably chloride, in a non reactive solvent such as a chlorinated hydrocarbon, methanol or tetrahydrofuran.

A compound of formula XIII or its ester, wherein X is guanidino or $-NHC(NH)R^6$ ($R^6$, R, M and A being as defined above) can be prepared by reacting the corresponding 4"-amino compound or its ester with S-methyl isothiourea or an imino ether(e.g.$R^6C(NH)OC_2H_5$) respectively. Preferably equivalent quantities of the reactants are used, the S-methyl isothiourea and iminoether, respectively being in ethanolic solution. Usually the reaction is carried out at room temperature. The product is isolated from the reaction mixture by procedures well known in the art.

A compound of formula XIII or its ester, wherein X is $-NH(CH_2)pOH$ or $-NH(CH_2)pOR^{10}$ wherein p is an integer of from 2 to 18, and $R^{10}$, R, M and A being as defined above, can be prepared by reacting the corresponding 4"-amino compound or its ester with hal-$(CH_2)pOH$ or hal-$(CH_2)pOR^{10}$($R^{10}$ and p being as defined above, hal representing halogen) in the presence of a base such as potassium carbonate in preferably ethanol at reflux temperature. The reaction mixture is diluted with water and the product is isolated by procedures well known in the art.

A compound of formula XIII (preferably its ester) wherein Y together with X represents oxo can be reduced to yield the corresponding 4"(α,β)-hydroxy compound. A suitable reducing agent is lithium tri-

tertiary butoxy aliminium hydride, $LiAlH[OC(CH_3)_3]_3$. Preferably this reaction is carried out in a non-reactive solvent such as tetrahydrofuran. If the starting material contains a 9-oxo group the corresponding 9(α,β)-dihydro derivative is obtained by this process. If for example a 2'-ester of 4"-dehydro antibiotic AR5-1 or 4"-dehydro antibiotic AR5-2 is subjected to this reaction a mixture of the isomers of the corresponding 9,4"-dihydroxy compounds is obtained.

If desired the so obtained product may be subjected to oxidation by means of $MnO_2$ to yield the corresponding 9-oxo-4"-hydroxy compounds.The epimeres may be separated from each other before or preferably after this oxidation.

A compound of formula XIII wherein X in combination with Y is $=N-NH_2$, $=N-NHR^{11}$ or $=NR^{11}$ can be prepared by reacting the corresponding compound of formula XIII (preferably its ester),wherein X in combination with Y is oxo, obtained by the above described process, with hydrazine, substituted hydrazine $R^{11}-NH-NH_2$ or a primary amine $R^{11}NH_2$ respectively. For example a 2'-ester of 4"-

dehydro antibiotic AR5-1 or of 4"-dehydro antibiotic AR5-2 can be converted to the 2'-ester of the corresponding Schiff base (X in combination with Y being $=NR^{11}$) or of the corresponding hydrazone (X in combination with Y being $=N-NHR^{11}$). $R^{11}$ is as defined above.

As can be seen from the above described processes compounds of formula XIII wherein X is mesyloxy, tosyloxy, halo, azido or amino and wherein X in combination with Y is oxo, are useful intermediates.

In several of the above described processes mixtures of isomers are obtained. These isomers can be separated by means known in the art such as chromatography. The separation can be applied to the free compounds or to their esters.

The compounds of this invention contain hydroxyl group(s) at positions 2' and/or 4" and/or 9 and/or 14 and/or 21, which can be esterified to form non-toxic pharmaceutically acceptable esters with hydrocarbon carboxylic acids. Typical acylating agents are the anhydrides, chlorides or activated esters of the hydrocarbon carboxylic acids. The esterification is usually carried out in a non-reactive

solvent such as acetone, tertiary amine (pyridine), tetrahydrofuran and dimethylformamide.

The reactivity of these hydroxy groups is different and consequently compounds containing free and esterified hydroxy groups or hydroxy groups esterified by different acids can be prepared by appropriate sequence of esterification and hydrolysis steps. (Hydrolysis can for example be carried out in lower alcohol, e.g. methanol). This is explained by the following examples:

The hydroxy group in positions 2' has the greatest reactivity. The 2'-monoesters may be prepared directly by treating the free unesterified antibiotic with a stoichiometric quantity (or a slight excess) of an acylating agent, such as an acyl-halide or an acyl anhydride in the absence of a base at about ambient temperature preferably in a non-reactive solvent such as acetone. The reaction is continued for from about 1 to about 20 hours until esterification is substantially complete and followed by isolation of the 2'-monoester from the reaction mixture.

The 2',4"-diesters may be prepared directly by treating the free antibiotics with an excess of

acylating agent at about ambient temperature for from about 1 to about 7 days until esterification is substantially complete and isolating the 2',4"-diester from the reaction mixture.

Alternatively, 2',4"-diesters may be prepared by treating a 2'-monoester with an excess of acylating agent under substantially the same conditions described above for direct esterification. This procedure is especially advantageous for preparing mixed esters wherein the desired compound has a different acyl function on each of positions 2' and 4". The 4"-monoester is conveniently prepared by the solvolysis of a 2',4"-diester.

The C-21 hydroxyl being a primary alcohol is esterifiable by acylating agents known in the art. However, in view of the greater reactivity of the 2'-hydroxyl in the desosamine moiety, C-21 esters are prepared by indirect methods. For example, by treating the desmycinosyl derivative with an excess of acylating agent such as acetic anhydride in a tertiary amine (pyridine), the 2',21-diester (e.g. 2',21-diacetate) is formed. To obtain the 21-monoester,

the 2',21-diester is subjected to selective solvolysis such as that described in U.S. Patent 4,056,616 issued November 1, 1977 whose disclosure is incorporated by reference herein.

In a compound containing hydroxy in positions 2',4" and 9 the 2'-hydroxy group has the highest reactivity. Mono-,di- and tri-esters can be prepared by appropriate sequence of esterification and hydrolysis steps.

If a compound containing a free amino group and one or more free hydroxy groups is subjected to acylation as described above, all these groups can be acylated (except the hydroxy group of position 14). In order to obtain a compound containing free hydroxy group(s) in position(s) 9 and/or 2' and/or 4" the product is subjected to selective hydrolysis by methods which take advantage

of the greater stability to hydrolysis of acylamino groups than the acylated hydroxy groups.

The esters of this invention are derived from carboxylic acids having 2 to 18 carbon atoms, generally used in the pharmaceutical arts.

Embraced by the term are aliphatic, cycloaliphatic, aromatic and heterocyclic including the hemi esters formed with dicarboxylic acids. Examples of suitable acids are acetic, propionic, valeric, stearic, tartaric, maleic, oxalic, malic, malonic, citric, oleic, palmitic, lauric, lactic, fumaric, succinic, cyclopropylcarboxylic, cyclopentylcarboxylic, adamantoic, furic, nicotinic, thenoic, picolinic, benzoic, phenylacetic acid and the like.

Pharmaceutically acceptable acid addition salts of this invention are derived from acids generally employed in the pharmaceutical art, including inorganic acids, such as for example sulfuric, phosphoric and hydrohalic (e.g. hydrochloric), and boric acid and carboxylic acids having 2 to 18 carbon atoms, such as exemplified above for the esters.

Acid addition salts have enhanced water solubility and are, therefore, useful for parenteral administration.

The following compounds exemplify the compounds of formula I and their esters, which can be prepared according to the described processes:

Antibiotic AR5-1,

antibiotic AR5-2

12,13-desepoxy-12,13-dehydro antibiotic AR5-1,

12,13-desepoxy-12,13-dehydro antibiotic AR5-2,

2'-acetyl antibiotic AR5-1,

2'-acetyl antibiotic AR5-2,

2'-acetyl-12,13-desepoxy-12,13-dehydro antibiotic AR5-2,

4"-acetyl antibiotic AR5-2,

4"-acetyl-12,13-desepoxy-12,13-dehydro antibiotic AR5-2,

2',4"-diacetyl antibiotic AR5-2,

2',4"-diacetyl-12,13-desepoxy-12,13-dehydro antibiotic AR5-2,

desmycinosyl-12,13-desepoxy-12,13-dehydro antibiotic AR5-1,

desmycinosyl-12,13-desepoxy-12,13-dehydro antibiotic AR5-2,

desmycinosyl-12,13-desepoxy-12,13-dehydro-21-acetyl and -21,2'-diacetyl antibiotic AR5-2,

desmycinosyl-12,13-desepoxy-12,13-dehydro-2'-acetyl antibiotic AR5-2,

11-amino-10,11-dihydro antibiotic AR5-1,

11-amino-10,11-dihydro antibiotic AR5-2,

11-ethylthio-10,11-dihydro antibiotic AR5-1,

11-ethylthio-10,11-dihydro antibiotic AR5-2,

11-(p-methoxybenzylthio)-10,11-dihydro antibiotic AR5-2,

2',4"-diacetyl-11-acetamido-10,11-dihydro antibiotic AR5-2,

2'-acetyl-11-acetamido-10,11-dihydro antibiotic AR5-2,

11-acetamido-10,11-dihydro antibiotic AR5-2,

11-(1-piperidinyl)-10,11-dihydro antibiotic AR5-2,

11-morpholino-10,11-dihydro antibiotic AR5-2,

11- 4-methylpiperazinyl-10,11-dihydro antibiotic AR5-2,

11-thiomorpholino-10,11-dihydro antibiotic AR5-2,

11-L-cysteinyl-ethyl ester-10,11-dihydro antibiotic AR5-2,

10,11-dihydro-11-(4-ethyl-2,3 dioxo-piperazinocarbonylamino)antibiotic AR5-1,

10,11-dihydro-11-(4-ethyl-2,3 dioxo-piperazinocarbonylamino) antibiotic AR5-2,

9,9,10,11-tetrahydro-11-(4-ethyl-2,3-dioxo-piperazinocarbonylamino) antibiotic AR5-1,

9,9,10,11-tetrahydro-11-(4-ethyl-2,3- dioxo-piperazinocarbonylamino)antibiotic AR5-2,

10,11-dihydro-11-(p-methoxybenzylthio)antibiotic AR5-1,

10,11-dihydro-11-(p-methoxybenzylthio)antibiotic AR5-2,

10,11-dihydro-11-(ethylthioxanthyl) antibiotic AR5-1,

10,11-dihydro-11-(ethylthioxanthyl)antibiotic AR5-2,

10,11-dihydro-11-(penicillaminyl)antibiotic AR5-1,

10,11-dihydro-11-(penicillaminyl)antibiotic AR5-2,

10,11-dihydro-11-(cysteinyl)antibiotic AR5-1,

10,11-dihydro-11-(cysteinyl)antibiotic AR5-2,

9-dihydro antibiotic AR5-1,

9-dihydro antibiotic AR5-2,

2'-acetyl-4"-dehydro-12,13-desepoxy-12,13-dehydro antibiotic AR5-2,

2'-acetyl-4"-dehydro antibiotic AR5-2,

2'-acetyl-4"-dehydro antibiotic AR5-1,

9-dihydro-12,13-desepoxy-12,13-dehydro antibiotic AR5-1,

2'-acetyl-4"-methanesulfonyl-12,13-desepoxy-12,13-dehydro antibiotic AR5-2,

9-dihydro-12,13-desepoxy-12,13-dehydro antibiotic AR5-2,

9,2',4"-triacetyl-9-dihydro-12,13-desepoxy-12,13-dehydro antibiotic AR5-2,

4"-methanesulfonyl-12,13-desepoxy-12,13-dehydro antibiotic AR5-2,

2'-acetyl-9-dihydro-12,13-desepoxy-12,13-dehydro antibiotic AR5-2,

10,11-dihydro antibiotic AR5-1

21-dimethylamino-desmycinosyl-12,13-desepoxy-12,13-dehydro antibiotic AR5-1,

21-dimethylamino-desmycinosyl-12,13-desepoxy-12,13-dehydro antibiotic AR5-2,

21-amino-desmycinosyl-12,13-desepoxy-12,13-dehydro antibiotic AR5-1,

21-amino-desmycinosyl-12,13-desepoxy-12,13-dehydro antibiotic AR5-2,

9-dihydro-21-amino-desmycinosyl-12,13-desepoxy-12,13-dehydro antibiotic AR5-1,

9-dihydro-21-amino-desmycinosyl-12,13-desepoxy-12,13-dehydro antibiotic AR5-2

21-oxo-desmycinosyl-12,13-desepoxy-12,13-dehydro antibiotic AR5-1

21-oxo-desmycinosyl-12,13-desepoxy-12,13-dehydro antibiotic AR5-2

9-dihydro-21-guanidino-desmycinosyl-12,13-desepoxy-12,13-dehydro antibiotic AR5-1

9-dihydro-21-guanidino-desmycinosyl-12,13-desepoxy-12,13-dehydro antibiotic AR5-2

9-dihydro-desmycinosyl-12,13-desepoxy-12,13-dehydro antibiotic AR5-1

9-dihydro-desmycinosyl-12,13-desepoxy-12,13-dehydro antibiotic AR5-2

Based upon classical chemical analyses such as nuclear magnetic resonance (NMR) spectrometry, infrared spectroscopy (IR), ultraviolet spectroscopy (UV), mass spectroscopy (MS) and also upon chromatography against known antibiotics, it was determined that the members of the AR-5 complex are macrolides. Hydrolysis of the individual components established the presence of two sugars, mycinose and desosamine. Both of the sugars have been found to be constituents of other well known antibiotics. However, they have not heretofore been found to be constituents of the same macrolide antibiotic and in the same positions.

Physical data for representative compounds of the invention are shown in the following tables 6 to 10.

TABLE 6

I Empirical Formulae, Molecular Weights:

| | | |
|---|---|---|
| AR-5 component 1 | $C_{37}H_{61}NO_{12}$ | MW 711 |
| AR-5 component 2 | $C_{37}H_{61}NO_{13}$ | MW 727 |
| 12,13-desepoxy-12,13-dehydro AR-5 component 1 | $C_{37}H_{61}NO_{11}$ | MW 695 |
| 12,13-desepoxy-12,13-dehydro AR-5 component 2 | $C_{37}H_{61}NO_{12}$ | MW 711 |

II Nuclear Magnetic Spectra[1]

| Proton | AR-5 component 1[2] | 12,13-desepoxy-12,13-dehydro AR-5 component 1[3] |
|---|---|---|
| =CH(2) | 5.88d(15.0) | 5.93 |
| =CH(11) CH- = (Four) | 6.55 | CH= (six) 6.18 |
| =CH(3) | 6.68 | 6.60 |
| | | 6.98 |
| =CH(10) | | 7.02 |
| $=CH_{15}$ | 5.36m | 4.97m |
| OCHO Mycinose | 4.59d (8.0) | 4.58d (8.0) |
| OCHO desosamine | 4.38d (7.0) | 4.30d (7.5) |
| OCHO | | |
| $OCH_3$ (two) | 3,58,363 singlets | 3.48, 3.54 singlets |
| CH | | |
| $N(CH_3)_2$ | 2.28s | 2.28s |
| $CH_2(16)$ | | |
| $CH_2(7)$ | | |
| $CH_3(4)$ | | 1.22d(7.0) |
| $CH_3$(three) | | 1.14d,1.14d,1.18d |
| $CH_3$ | 1.02d(6.5) | 1.00d(6.5) |
| $CH_3(C-16CH_3)$ | 0.88t(7.0) | 0.92t(7.0) |

1 100 MHz Proton NMR

2 Solvent $(CD_3)_2CO$

3 Solvent $CDCl_3$

TABLE 7

| Proton | AR-5 component 2[4)] |
|---|---|
| =CH(2) | 6.96d(15.0) |
| =CH(11) CH- (Four) | 6.66d(15.0, 10.0) |
| =CH(3) | 6.38dd(15.0, 9.0) |
| =CH(10) | 6.09d(15.0) |
| $=CH_{15}$ | 5.44d(8.5, 5.5) |
| OCHO Mycinose | 4.62d(8.0) |
| OCHO desosamine | 4.32d(7.0) |
| OCHO | 3.84 |
| | 3.76 |
| $OCH_3$ (two) | 3.56S, 3,56S |
| CH | 3.18 |
| | 2.50 |
| $N(CH_3)_2$ | 2.28 |
| $CH_2(16)$ | 1.80m |
| $CH_2(7)$ | 1.70m |
| $CH_3(4)$ | 1.26d(7.0) |
| $CH_3$(three) | 1.18d(6.5) |
| $CH_3$ | 1.04d(6.5) |
| $CH_3(C-16CH_3)$ | 0.86t(7.0) |

4) Solvent $CDCl_3$

TABLE 8

Ultraviolet

Antibiotic AR-5 Component 1

max

217 nm $E^{1\%}$ = 329 ( = 23,400)

245 $E^{1\%}$ = 170 ( = 12,070)

Antibiotic AR-5 Component 2

max $[\alpha]^{26°C}$ = 22.9 ($C_2H_5OH$)

216 nm $E^{1\%}$ = 324 ( = 23,390)

246 nm $E^{1\%}$ = 138 ( = 10,000)

12,13-desepoxy-12,13-dehydro AR-5 Component 1

max $[\alpha]^{26°C}$ = 7.2 ($C_2H_5OH$)

214 nm $E^{1\%}$ = 290 ( = 20,160)

280 nm $E^{1\%}$ = 295 ( - 20,500)

12,13-desepoxy-12,13-dehydro AR-5 Component 2

max $[\alpha]^{26}$ = 68.1 ($CH\ Cl_3$;0.3%)

214 nm = (20,067)

278 nm - (19,702)

TABLE 9

COMPARATIVE THIN LAYER CHROMATOGRAPHY OF ANTIBIOTIC AR-5 COMPLEX WITH SOME MACROLIDE ANTIBIOTICS

| System | Antibiotic | $R_f$ of Inhibition Zone |
|---|---|---|
| Chloroform-Methanol-17% Ammonia 2:1:1 | Antibiotic AR-5 Complex | 0.98 |
| | Rosaramicin | 0.98 |
| | Megalomicin | 0.97 |
| | Oleandomycin | 0.95 |
| | Erythromycin | 0.94 |
| | Spiramycin | 0.95 |
| | Magnamycin | 0.96 |
| Butanol-Acetic Acid-Water-Dioxane 6:2:2:1 | Antibiotic AR-5 Complex | 0.5 |
| | Rosaramicin | 0.41 |
| | Oleandomycin | 0.39 |
| | Erythromycin | 0.45 |
| | Spiramycin | 0.32 |
| | Magnamycin | 0.59 |
| Chloroform-Methanol-Petroleum Ether-Water 3:3:1:1 | Antibiotic AR-5 component 1 | 0.51 |
| | Antibiotic AR-5 component 2 | 0.43 |
| | Rosaramicin | 0.40 |
| | Oleandomycin | 0.34 |
| | Erythromycin | 0.34 |
| | Spiramycin | 0.53 |
| | | 0.58 |
| | Magnamycin | 0.75 |
| Butanol-Acetic Acid-Water 3:1:1 | Antibiotic AR-5 Complex | 0.46 |
| | Rosaramicin | 0.41 |
| | Megalomicin | 0.38 |
| | Oleandomycin | 0.41 |
| | Erythromycin | 0.48 |
| | Spiramycin | 0.23 |
| | Magnamycin | 0.59 |

In the foregoing Chromatographic Comparisons it should be noted that none of the systems completely separates the antibiotics of this invention from each other. They do, however, serve to distinguish the Antibiotic AR-5 Complex from some of the known macrolides.

TABLE 10

CHARACTERISTIC INFRARED ABSORPTION PEAKS

The characteristic infrared absorption peaks are set forth as wave number values and are accurate within 3 $cm^{-1}$. The spectra were obtained on a Perkin-Elmer Model 180 I.R. grating spectophotometer as solutions in chloroform.

The legends (letters) set forth have the following meanings: s = strong; m = medium; w = weak and vs = very strong.

TABLE 10

| Antibiotic AR-5 Component 1 | | Antibiotic AR-5 Component 2 | | 12,13-desepoxy-12,13-dehydro - AR-5 Component 1 | | 12,13-desepoxy-12,13-dehydro - AR-5 Component 2 | |
|---|---|---|---|---|---|---|---|
| 3540 $cm^{-1}$ | (m) | 3540 $cm^{-1}$ | (m) | 3540 $cm^{-1}$ | (m) | 3550 | (m) |
| 3420 | (m) | 3430 | (m) | 3420 | (m) | 3420 | (m) |
| 2975 | (m) | 2980 | (m) | 2975 | (m) | 2980 | (m) |
| 2940 | (m) | 2940 | (m) | 2940 | (m) | 2940 | (m) |
| 2885 | (m) | 2885 | (m) | 2885 | (m) | 2880 | (m) |
| 2790 | (m) | 2795 | (m) | 2795 | (m) | 2835 | (m) |
| 1710 | (m) | 1715 | (m) | 1710 | (m) | 2790 | (m) |
| 1690 | (m) | 1715 | (m) | 1675 | (m) | 1710 | (m) |
| 1650 | (m) | 1690 | (m) | 1650 | (m) | 1680 | (m) |
| 1675 | (m) | 1655 | (m) | 1630 | (m) | 1650 | (m) |
| 1595 | (m) | 1630 | (m) | 1595 | (m) | 1635 | (m) (shoulder) |
| 1235 | (m) | 1610 (shoulder | (m)) | 1230 | (m) | 1595 | (m) |
| 1220 (shoulder | (m)) | 1240 | (m) | 1225 (shoulder | (m)) | 1235 | (m) |
| 1165 | (s) | 1230 (shoulder | (m)) | 1165 | (s) | 1225 | (m) (shoulder) |
| 1110 | (s) | 1175 | (s) | 1110 | (s) | 1170 | (s) |
| 1095 (shoulder | (m)) | 1110 | (s) | 1050 (VS, | | 1110 | (s) |
| 1072 | (vs) | 1085 | (vs) | 1050 broad) | | 1095 | (s) (shoulder) |
| 1050 (shoulder | (vs)) | 1060 | (vs) | 1000 | (m) | 1072 | (vs) |
| 985 | (s) | 990 | (s) | 985 | (s) | 1050 | (vs) (shoulder) |
| 885 | (m) | 890 | (m) | 885 | (w) | 1000 | (m) |
| 860 | (m) | 865 | (m) | 860 | (w) | 985 | (s) |
| 830 | (m) | 840 | (m) | 840 | (w) | 910 | (w) |
| | | | | | | 885 | (w) |
| | | | | | | 860 | (w) |
| | | | | | | 845 | (m) |
| | | | | | | 835 | (m) |

On the basis of the foregoing physicochemical data and the isolation of mycinose and desosamine from the hydrolysis of the respective antibiotic compounds, it is concluded that antibiotic AR5-1, antibiotic AR5-2 and the corresponding 12,13-dehydro derivatives (defined above) have the planar structural formula I. The group of formula II has the configuration of D-mycinose. The same planar formula applies to the derivatives of these compounds disclosed herein.

The antibiotics of this invention were subjected to art recognized tests to determine their antibacterial profile. The test methodology and a summary of the results therefrom are set forth hereinbelow.

Antibiotic AR5-1, antibiotic AR5-2, their 12,13-desepoxy-12,13-dehydro analogs, erythromicin and rosaramicin have been subjected to the same tests:

In vitro broth dilution tests (MIC's) were done in conventional manner using either Mueller-Hinton broth (gram-positive and gram-negative bacteria), Sabouraud Dextrose Broth (fungi) or Fluid Thioglycollate broth (anaerobic bacteria) at the appropriate pH, in volumes of 3-5 ml/tube. Inocula were obtained by diluting either overnight cultures (gram-positive and gram-negative

bacteria and yeasts), 48 hr. cultures (anaerobic bacteria), or 4-5 day cultures (dermatophytes). Tubes were incubated at 28°C (dermatophytes), or 37°C (gram-positive and gram-negative bacteria and yeasts), and endpoints were read after 24, 48 and 72 hours.

In vitro agar dilution tests were performed using Mueller-Hinton agar which was melted prior to use and cooled to approximately 55°C before the addition of suitable volumes of compound solution, to provide twofold serial dilutions. Thirty ml was transferred to 100 x 15 mm square "Integrid" petri dishes and allowed to dry overnight. Plates were inoculated with a Steers-Foltz replicating device. Inoculum concentrations were adjusted so that each inoculating rod of the Steers' replicator delivered approximately $10^4$ viable units of each organism to the agar surface. Inoculated plates were allowed to dry and then incubated at 37°C. MIC's were determined after 24 and 48 hours of incubation. The MIC was the lowest concentration at which less than six isolates, discrete colonies were visible.

In the following tables 11-13, the tested antibiotics are set forth in the following order: antibiotic AR5-1 is A, 12,13-desepoxy-12,13-dehydro antibiotic AR5-1 is B, antibiotic AR5-2 is C, 12,13-desepoxy-12,13-dehydro

antibiotic AR5-2 is D, Erythromycin is E and Rosaramicin is F:

TABLE 11

ANTIBACTERIAL MEAN MIC's OF SELECTED MACROLIDES

| Organism | No. of Isolates | A | B | C | D | E | F |
|---|---|---|---|---|---|---|---|
| *S. lutea* | 1 | .06 | .06 | .06 | <.04 | .125 | .06 |
| *B. subtilis* | 2 | .09 | .17 | .18 | .25 | .35 | .71 |
| *Staph.* $E^SR^S$ | 13 | .075 | .080 | .123 | | .06 | .139 |
| $E^RR^S$ | 3 | .2 | 0.5 | 0.63 | | 256 | 2.5 |
| $E^RR^R$ | 5 | 111 | 111 | 194 | | 256 | 256 |
| Enterococcus | 4 | 14 | 19 | 32 | 4 | 0.5 | 0.8 |
| *C. albicans* | 2 | 256 | 256 | 256 | >32 | 256 | 256 |
| Enterobacter | 5 | 194 | 223 | 128 | >32 | 147 | 16 |
| *E. coli* | 13 | 55 | 75 | 25 | 16 | 22 | 5 |
| Klebsiella | 12 | 181 | 192 | 81 | >32 | 68 | 15 |
| Providencia/proteus (indole positive) | 12 | 192 | 203 | 144 | >32 | 228 | 31 |
| Pseudomonas | 20 | 187 | 187 | 187 | >32 | 158 | 74 |
| Salmonella | 5 | 223 | 256 | 147 | >32 | 97 | 11 |
| Shigella | 1 | 32 | 128 | 64 | 8 | 32 | 4 |
| Serratia | 9 | 220 | 256 | 188 | >32 | 138 | 19 |

The Staphylococcus isolates were divided into three groups: $E^SR^S$, erythromycin and rosaramicin sensitive strains, $E^RR^S$, erythromycin resistant and rosaramicin sensitive strains, $E^RR^R$, strains resistant to both erythromycin and rosaramicin. Only against the final group of macrolide resistant strains did the antibiotics of this invention lose activity. They were active against some anaerobe strains such as B. fragilis and Clostrida but did not show the gram-negative potency of rosaramicin.

In vivo mouse protection tests were performed in male (Carworth CF-1) mice weighing 18-20 g each. Infection was effected, with about $10^7$ organisms per mouse or sufficient inocula to cause death of control groups 24 to 48 hours after infection. The infecting organisms included strains of Staphylococcus Enterococcus, β-hemolytic Streptococcus and E.coli.

The serum levels of the respective antibiotics were measured in mice, rats and dogs. The results were surprising in that the novel antibiotics exhibit therapeutic serum levels considerably longer than erythromycin or rosaramicin. It was also observed that antibiotic AR5-2 produces peak serum levels earlier than any of the other antibiotics tested,

however, antibiotic AR5-1 produces therapeutic serum levels of greater duration. This phenomenon gives substantial support for pharmaceutical dosage forms containing both compounds. This would provide for fewer doses per day without sacrificing the therapeutic effect.

TABLE 12

Mean $PD_{50}$'s

Antibiotic AR-5 Sensitive (mg/kg)

| Route | A | B | C | D | E | F |
|---|---|---|---|---|---|---|
| s.c. | 4.0(7)* | 2.5(7) | 5.1(7) | -- | 13.3(6) | 8.3(6) |
| oral | 4.1(7) | 3.8(7) | 35.8(7) | -- | 135.0(6) | 181.0(6) |
| | | | Antibiotic AR-5 Resistant | | | |
| s.c. | 100(5) | 100(5) | 100(5) | | 100(5) | 100(5) |
| oral | 100(5) | 100(5) | 100(5) | | 400(5) | 400(5) |

*Number of strains averaged is given in parentheses,

Comparative *in vivo* $PD_{50}$'s with erythromycin and rosaramicin demonstrated a major advantage that the compounds of this invention offer. Unlike erythromycin and rosaramicin, with one exception, the compounds of this invention appear to have equal potencies orally and subcutaneously. In general antibiotic AR5-1 and 12,13-desepoxy-12,13-dehydro antibiotic AR5-1 were 30-40 fold more potent orally than erythromycin and rosaramicin.

The acute toxicity determinations were carried out with groups of male (Carworth CF-1) mice weighing 18-20 g. $LD_{50}$ values (the dose that was lethal to 50% of the mice) were calculated by probit analysis based on the number of survivors at 24 and 48 hours after dosing.

TABLE 13

$LD_{50}$'s (mg/kg)

| Route | A | B | C | D | E | F |
|---|---|---|---|---|---|---|
| i.v. | 140 | 160 | 310 | --- | 260 | 175 |
| i.p. | 310 | 275 | 610 | --- | 355 | 260 |
| oral | 2000 | --- | 2000 | --- | --- | 1300 |

The following table 14 summarizes data (MIC) showing the activity of representative macrolide antibiotics of this invention which are derivatives derived from the compounds of antibiotic AR5-complex.

The data have been obtained in Mueller Hinton Agar after 24 hours of incubation as described on page 81 lines 6 to 20 herein before.

Antibacterial mean MIC's of selected macrolides

| Name | I | II | III |
|---|---|---|---|
| 9-dihydro-12,13-desepoxy-12,13-dehydro antibiotic AR5-2 | 0.77 | $\geq$2.0 | |
| 9-dihydro-2'-acetyl-12,13-desepoxy-12,13-dehydro antibiotic AR5-2 | 1.1 | 2.5 | |
| 2'-acetyl-4''-dehydro antibiotic AR5-2 | 1.35 | 3.5 | 8 |
| 11-thiomorpholino-10,11-dihydro antibiotic AR5-2 | 0.32 | 0.63 | 16 |
| 11-L-cysteinyl-ethyl ester-10,11-dihydro antibiotic AR5-2 | 0.31 | 0.84 | 22.6 |
| 11-(1-piperidinyl)-10,11-dihydro antibiotic AR5-2 | 0.17 | 0.63 | 16 |
| 10,11-dihydro-11-L-ethylthio antibiotic AR5-2 | 0.26 | 0.54 | 3.6 |
| 10,11-dihydro-11-L-ethylthio antibiotic AR5-1 | 0.15 | 0.43 | 5.9 |
| 11-amino-10,11-dihydro antibiotic AR5-2 | $\leq$0.095 | 0.28 | 1.26 |
| 11-amino-10,11-dihydro antibiotic AR5-1 | 0.14 | 0.08 | 0.76 |
| 10,11-dihydro-11-L-(p-methoxybenzylthio)antibiotic AR5-2 | 0.14 | 0.27 | 1.0 |
| Desmycinosyl-12,13-desepoxy-12,13-dehydro antibiotic AR5-1 | 3.4 | 7.1 | 2.0 |

I: geometric means of results against strains of B. Subtilis (2 strains), Sarcine lutea (1 strain), Staph. aureus (13 strains)

II: geometric means of results against 12 strains of β-hemolytic Strep.groups A, C and G

III: geometric means of results against 8 strains of Strep. pneumoniae

The compounds of this invention exhibit an antibacterial effect against a wide variety of bacterial species, especially against strains of gram-positive bacteria. Exemplary of the bacteria against which the compounds of this invention are active are various strains of Streptococcus pyogenes, Staphylococcus aureus and Bacillus subtilis. The compounds may thus be used in the treatment of warm-blooded animals suffering from diseases caused by such organisms. The absorption is better than of similar macrolide antibiotics. The compounds may also be used for in vitro purposes as disinfectants for laboratory glassware, dental and medical equipment.

The antibiotics of this invention may be administered topically, parenterally and orally, preferably in admixture with suitable pharmaceutical carriers and excipients.

The dosage forms, excluding topicals, should be designed to permit the administration of from about 5 to about 50 mg per kg per day. Topical formulations should be applied to the affected areas from about 2 to about 4 times a day and should contain from about 5 to 15, preferably about 10, grams per liter for lotions and the same quantity per kilogram for ointments.

However, it should be realized that many factors such as for example the age and general physical condition of the patient, the nature of the infecting organism influence the precise dosage of a medicament to be administered.

The antibacterial compounds of this invention may be dispensed in the form of for example capsules, tablets, elixirs and as injectable solutions and suspensions.

Formulations may contain one or more of the antibiotics of this invention.

In tests with mamals it has been determined that antibiotic AR5-2 reaches a peak serum level earlier than antibiotic AR5-1 by subcutaneous or oral routes, whereas antibiotic AR5-1 gives peak serum levels which persist substantially longer than those of antibiotic AR5-2. Thus a combination dosage has the advantage of an early and prolonged peak serum level.

A preferred combination is a pharmaceutical dosage form containing 30 to 60% preferably 40 to 50% of one of antibiotic AR5-1 and antibiotic AR5-2 in dosage form in admixture with suitable pharmaceutical excipients, wherein the remainder of the antibacterially active

material is the other of antibiotic AR5-1 and antibiotic AR5-2. Another preferred formulation contains the said antibiotics in equal parts.

It can be concluded from test results that the compounds of this invention are useful anti-bacterial agents. Certain groups and individual compounds are of particular interest:

-) From the esters of the compounds of formula I those are preferred wherein the hydroxy group at position 9 and/or 21 and/or 2' and/or 4" is/are esterified with mono-carboxylic acid(s) or di-carboxylic acid(s) each having 2 to 18 carbon atoms, especially those wherein the hydroxy group(s) is(are) esterified with a mono-carboxylic acid containing 2 or 3 carbon atoms.

-) From the compounds of formula I containing substituents $R^2$ and/or $R^3$ and/or $R^4$ and/or $R^5$ and/or $R^6$ and/or $R^7$ and/or $R^8$ and/or $R^{10}$ and/or $R^{11}$ those are preferred wherein $R^2$, $R^3$, $R^4$, $R^6$, $R^7$, $R^{10}$ and $R^{11}$ are an alkyl group containing straight or branched chain alkyl radicals having 1 to 8 carbon atoms, preferably being methyl or ethyl, $R^5$ is hydrogen and $R^8$ is phenyl.

-) The term "heterocycle" as used in the definition of substituents $R^2$, $R^3$, $R^9$ and $R^{11}$ can be exemplified by pyridyl, furanyl, pyrrolidinyl, piperazinyl, pyrazinyl, azetidinyl, tetrazole, triazole, oxazole and isoxazole.

-) The term amino "acid residue" as used in the definition of the compounds of formula I can be exemplified by alanyl, glycyl, arginyl, sarcosyl, tyrosyl, phenylalanyl, methionyl, seryl, lysyl, asparaginyl, isoleucyl, leucyl, threonyl, histidyl, aspartyl, valyl, prolyl, glutaminyl, triptophanyl and glutamyl.

-) Compounds of the general formula XIV,

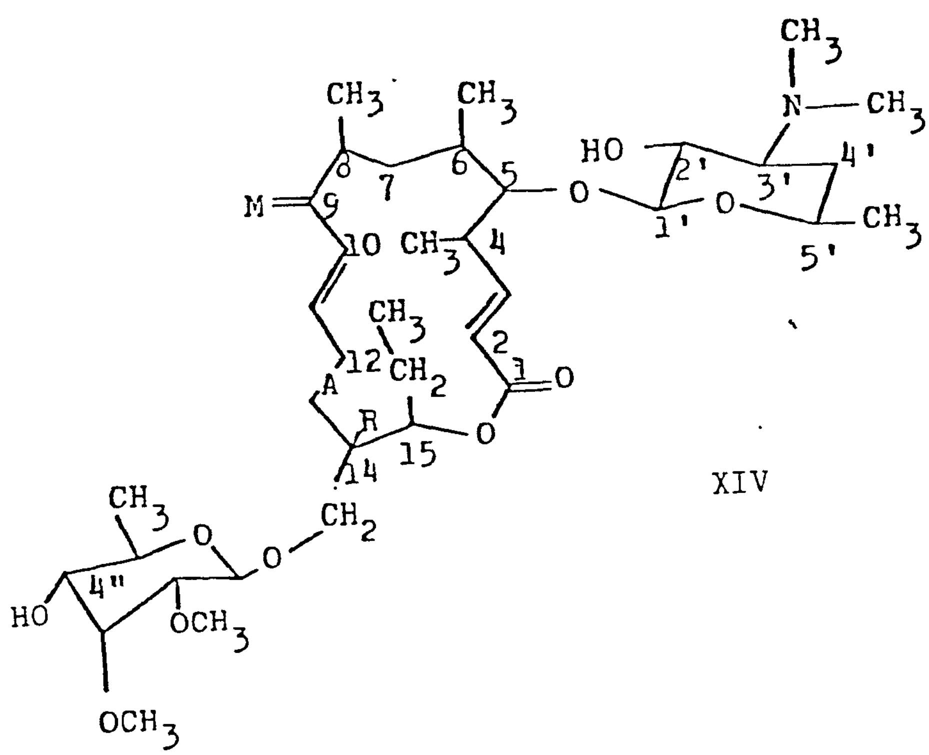

XIV

in the free form or in the form of its ester or salt, wherein R is hydrogen or hydroxy, M is oxo or (H,OH), A together with the carbon atoms of positions 12 and 13 represents an oxirane ring, or a double bond between the carbon atoms of positions 12 and 13, the compounds being antibiotic AR5-1, antibiotic AR5-2, their 9(α,β)-(H,OH)- analogs and the 12,13-desepoxy-12,13-dehydro derivatives of these compounds. As mentioned before, a mixture of antibiotic AR5-1 and antibiotic AR5-2 is particularly preferred, wherein antibiotic AR5-1 constitutes 30 to 60 percent (preferably 50 percent) of the mixture, antibiotic AR5-2 constituting the remainder of the mixture.

-) Compounds of formula I in the free form or in the form of their esters or salts, wherein R is hydrogen or hydroxy, M is oxo or (H,OH), A and B represent double bonds and Z is $-CH_2OH$, $-CHO$, $-CH_2NH_2$, $-CH_2NHR^2$, $-CH_2NR^2R^3$, $-CH_2OCOR^2$, $-CH_2NHCOR^2$, $-CH_2NR^3COR^2$,

$-CH_2NH-C(=NH)-R^2$, $-CH_2NHC(=NH)-NHR^5$, $-CH_2NHCO-$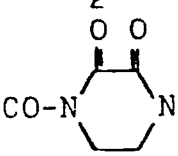$-CH_2CH_3$,

$-CH_2O$-glycosyl excluding $-CH_2O$-mycinosyl and $-CH_2O$-(3"-desmethyl-mycinosyl), $-CH_2$-ureido or $CH_2$-thioureido; and $R^2$, $R^3$ and $R^5$ are defined as above. Of these compounds, those are of particular interest wherein M is (H,OH) and those wherein Z is $-CH_2NH_2$, $-CH_2N(CH_3)_2$,

$-CH_2NHC(=NH)-NH_2$ and in particular $-CH_2OH$. Desmycinosyl-12,13-desepoxy-12,13-dehydro antibiotic AR5-1 is of great interest.

-) Compounds of the general formula I, in the free form or in the form of their esters or salts, wherein A together with the carbon atoms of positions 12 and 13 represents an oxirane ring, X is OH and Y is H, B together with the carbon atoms of positions 10 and 11 is the group $-CH_2-CH(Q^1)-$, wherein $-Q^1$ is $-NH_2$, $-NHC(O)R^6$, $-NHR^6$, $-NR^6R^7$, -NH-C(O)- 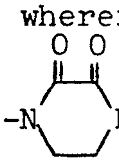 $-CH_2CH_3$, an amino acid residue, an esterified amino acid residue or a heterocyclic radical of 6 members containing either nitrogen as the only heteroatom or nitrogen and a second heteroatom which is nitrogen, oxygen, or sulfur; especially those, wherein $Q^1$ is $-NH_2$, $-NHC(O)R^6$, thiomorpholino, 4-methylpiperazinyl , morpholino or piperidinyl; R, M, $R^6$ and $R^7$ being defined as above. 11-Thiomorpholino-10,11-dihydro antibiotic AR5-2, 11-amino-10,11-dihydro antibiotic AR5-1, 11-amino-10,11-dihydro antibiotic AR5-2, 11-acetamido-10,11-dihydro antibiotic AR5-2, 11—(piperidinyl)-10,11-dihydro antibiotic AR5-2, 11-morpholino-10,11-dihydro antibiotic AR5-2, or 11-(4-methylpiperazinyl)——10,11-dihydro antibiotic AR5-2 are of particular interest.

-) 10,11-Dihydro antibiotic AR5-1.

-) Compounds of the general formula I in the free form or in the form of their esters or salts wherein A together with the carbon atoms of positions 12 and 13 represents an oxirane ring, X is OH and Y is H, B together with the carbon atoms of positions 10 and 11 is the group $-CH_2-CH(Q^2)-$, wherein $-Q^2$ is $-S-R^6$, thioxanthyl, a sulfur containing amino acid residue, or a sulfur containing esterified amino acid residue; and R, M, and $R^6$ are as defined above, especially 11-ethylthio-10,11-dihydro antibiotic AR5-1, 11-ethylthio-10,11-dihydro antibiotic AR5-2, 11-(p-methoxybenzylthio)-10,11-dihydro antibiotic AR5-2, or 11-L-cysteinyl-ethyl ester-10,11-dihydro antibiotic AR5-2.

-) Compounds of the general formula I, in the free form or in the form of their esters or salts, wherein X is hydrogen, OH, mesyloxy, halo, azido, amino, guanidino, $-NHR^6$, $-NR^6R^7$, $-NHR^8$, $-NHC(O)OR^6$, $-NHC(O)OR^8$ when Y is hydrogen; or Y in combination with X is oxo and R, M, $R^6$, $R^7$ and $R^8$ are as defined above, and A represents a double bond between the carbon atoms of positions 12 and 13 or, together with the carbon atoms of positions 12 and 13, an oxirane ring; B represents a double bond between the carbon atoms of positions 10 and 11,

especially those wherein X is mesyloxy and Y is hydrogen or Y in combination with X is oxo. 2'-Acetyl-4"-dehydro antibiotic AR5-2, 2'-acetyl-4"-dehydro antibiotic AR5-1, 2'-acetyl-4"-methanesulfonyl-12,13-desepoxy-12,13-dehydro antibiotic AR5-2, and 4"-methanesulfonyl-12,13-desepoxy-12,13-dehydro antibiotic AR5-2 being of particular interest.

Example 1

Preparation of the antibiotic AR-5 complex

Inoculum Preparation

Prepare 3.0 liters of second stage inoculum according to the procedure set forth above in the description, using *M.polytrota* NRRL 12066.

Fermentation

Six 14 liter fermenters containing 10 liters of Medium C are sterilized, cooled to 30°C and inoculated with 500 ml of second stage inoculum. Incubate each fermentation mixture at 30°C with rotary agitation at 350 rpm and aeration at 0.35 vvm. Adjust the fermentation mixture to pH 7 at the commencement of the fermentation and maintain at pH 7.0 ± 0.5 by adding dilute alkali as required. Continue the fermentation for about 72 hours, then commence monitoring for antibiotic production.

Isolation and purification of the antibiotics

When peak antibiotic concentration is attained, combine the six batches to form one 60 liter batch.

Extract the combined batches two times with 120 liters of ethyl acetate. Concentrate the extracts to an oil in vacuo. Absorb the oily residue on a column containing 2.1 liters of Sephadex LH-20 and elute with ethyl alcohol. Eluates containing antibacterially active fractions as determined by disc testing against Staphylococcus aureus 209P are combined, concentrated and absorbed onto a column containing 400 g of silica gel. Elute with the lower phase of a chloroform: methanol: petroleum ether: water system (3:3:1:1). Monitor the eluate using HPTLC and combine fractions containing like materials.

Yield - 913 mg antibiotic AR5-1 and 12,13-desepoxy-12,13-dehydro antibiotic AR5-1.

950 mg- antibiotic AR5-2 + 12,13-desepoxy-12,13-dehydro antibiotic AR5-2.

Purification of the antibiotics

Absorb 1.1 g of crude antibiotic AR5-2 (contaminated with 12,13-desepoxy-12,13-dehydro antibiotic AR5-2) on a column containing 130 g of silica gel and elute as previously described to obtain thereby 297 mg of antibiotic AR5-2 (HPLC Analysis shows that the

product is greater than 95% pure). Combine and concentrate the remaining eluates to obtain thereby 12,13-desepoxy-12,13-dehydro antibiotic AR5-2.

Repeat the above purification using 1.2 g of crude antibiotic AR5-1 (contaminated with 12,13-desepoxy-12,13-dehydro antibiotic AR5-1) to obtain 400 mg of an oily, unresolved mixture. Subject 8.9 mg of this mixture to preparative HPLC using Partial M9 PAC as the stationery phase and using the lower phase of a methylene chloride: heptane: water (3:2:1) system to obtain 7.5 mg of 95% pure by HPLC. Combine the remaining eluates to obtain thereby 12,13-desepoxy-12,13-dehydro antibiotic AR5-1.

Example 2

10,11-Dihydro-11——(amino)-antibiotic AR5-1

Dissolve 3.5 g of antibiotic AR5-1 in 35 ml of methanol saturated with ammonia. Stir the solution at room temperature (20°C) for 2 days and concentrate the solution to a residue. Dissolve the residue in a solvent system consisting of toluene, methylene chloride, methanol and concentrated ammonium hydroxide in the ratio of 16:4:1:1 and chromatograph on a silica gel column containing 400 g of silica gel

using the above described solvent system as eluant. Combine and concentrate the fractions containing the desired antibiotic to a residue. By substituting an equivalent quantity of antibiotic AR5-2 and by following the process of this Example, 10,11-dihydro-11——amino antibiotic AR5-2 is prepared.

Example 3

10,11-Dihydro-11——(p-methoxybenzylthio) antibiotic AR5-2.

Dissolve 100 mg of antibiotic AR5-2 in just enough tetrahydrofuran to dissolve it. Add 3 drops of p-methoxybenzylthiol and allow the solution to stand at room temperature ($20^{o}$) for about 72 hours. Dilute the reaction mixture with ethyl acetate. Chromatograph on silica gel GF. A preparative plates $1000\mu$ thick using as the developing solution a mixture of chloroform, methanol, petroleum ether, and water in the ratio of 3:3:1:1. Remove the silica gel containing the antibiotic, as determined by observing under ultraviolet light, and dissolve the product in ethyl acetate. Concentrate the ethyl acetate extract to a residue to obtain thereby 34 mg of antibiotic AR5-2. Extract the silica gel with acetone and concentrate to a residue yield-44 mg of the product of this example.

By substituting an equivalent quantity of antibiotic AR5-1 and following the process of this Example 10,11-dihydro 11 ——(p-methoxybenzylthio antibiotic AR5-1 is obtained.

Formulation 1

Capsule

| | |
|---|---|
| Antibiotic AR5-1 | 250.00 mg |
| Lactose | 248.75 mg |
| Magnesium Stearate | 1.25 mg |
| | 500.00 mg |

Procedure

1. Blend the antibiotic and the lactose.
2. Add the magnesium stearate and mix.
3. Fill capsule.

Formulation 2

Oral Suspension (to give a dose of 125 mg/5 ml)

| | |
|---|---|
| Antibiotic AR5-2 | 25.00 gms |
| Magnesium Aluminium Silicate | 9.50 gms |
| Sodium Carboxymethylcellulose U.S.P. | 2.50 gms |
| Flavor | qs |
| Color | qs |
| Methylparaben, U.S.P. | 0.90 gms |
| Propylparaben, U.S.P. | 0.20 gms |
| Polysorbate 80, U.S.P. | 1.00 gms |
| Sorbitol Solution, U.S.P. | 500.00 gms |
| Water, q.s. | 1000.00 ml |

Procedure

1. Heat 200 ml. of water to boiling, and dissolve in it one half of the parabens. Cool to about 70°C, then mix in the Polysorbate 80. Sprinkle in the silicate, stirring until a uniform smooth suspension results.

2. Heat an additional 200 ml. of water to boiling, and dissolve in it the remainder of the parabens. Disperse the CMC in this until a smooth gel results. Mix in the Sorbitol Solution. Then dissolve the sodium citrate therein.

3. Add the product of Step 2 to that of Step 1 slowly, with constant stirring. Cool the mixture to 25°C. Add the antibiotic AR-5 component 2, tartrate flavor, and color mixing thoroughly. Add sufficient quantity of water to make the total volume 1000 ml.

Formulation 3

Topical Ointment

| | |
|---|---|
| 12,13-desepoxy-12,13-dehydro antibiotic AR5-1 | 10 gms |
| Petrolatum | 990 gms |
| | 1000 gms |

Procedure

1. Melt the petrolatum.

2. Slurry the antibiotic with about 10% of the petrolatum and pass through a colloid mill.

3. Mix the milled slurry with the remainder of the molten petrolatum. Allow to cool.

Formulation 4

Topical Cream

| | |
|---|---|
| 12,13-desepoxy-12,13-dehydro antibiotic AR5-2 | 10 gms |
| Stearic Acid | 200 gms |
| Sorbitan Monostearate | 104 gms |
| Sorbitan Monooleate | 20 gms |
| Polyoxyethylene Sorbitan Monolaurate | 56 gms |
| Water, qs | 100 ml |

Procedure

1. Heat the stearic acid, sorbitan monostearate, sorbitan monooleate, and polyoxyethylene sorbitan monolaurate to 65°C.

2. Heat about 90% of the water to 70°C.

3. Add the water to Step 1 and mix to form a cream base.

4. Slurry the antibiotic with about 10% of the water

and pass through a colloid mill.

5. Add the milled slurry to the molten base and mix. Allow to cool.

Formulation 5

Capsule

| | |
|---|---|
| Antibiotic AR5-1 | 125 mg |
| Antibiotic AR5-2 | 125 mg |
| Lactose | 248.75 mg |
| Magnesium Stearate | 1.25 mg |
| | 500,00 mg |

Procedure

1. Blend the antibiotics and the lactose.

2. Add the magnesium stearate and mix.

3. Fill capsule.

In the foregoing formulations, the antibiotics of this invention, the non-toxic, pharmaceutically acceptable esters and the non-toxic, pharmaceutically acceptable acid addition salts thereof may be used, in equivalent quantity, interchangeably without substantially modifying the therapeutic effect to be derived from their use.

Example 4

2'-Acetyl-4"-dehydro antibiotic AR5-2

Dissolve 3g of AR5-2 in 70 ml of acetone at 20° to 25°C, add 0.4 ml of acetic anhydride with stirring and continue stirring for 6 hours. Add 30 ml of benzene and evaporate under high vacuum to give a white residue containing 2'-acetyl AR5-2.

Dissolve 3.6 g of 2'-acetyl AR5-2 in 13.7 ml of dry dimethylsulfoxide, 27 ml of dry benzene, and 0.33 ml of pyridine, 0.17 ml of freshly distilled trifluoroacetic acid and 2.54 g of dicyclohexylcarbodiimide. Stir the mixture at 20° to 25°C for 7 hours. Filter, wash the solids with benzene, combine the filtrate and washes and wash with 3x100 ml of distilled water then once with a saturated sodium chloride solution. Dry the organic layer over magnesium sulfate and evaporate the solvent to give 2'-acetyl-4"-dehydro AR5-2.

Example 5

Desmycinosyl-12,13-desepoxy-12,13-dehydro antibiotic AR5-1

Dissolve 3.5 g of 2'-acetyl-4"-dehydro-12,13-desepoxy-12,13-dehydro antibiotic AR5-1 (obtained by subjecting 12,13-desepoxy-12,13-dehydro antibiotic AR5-1 to the

procedure described in Example 4) in 100 ml of methanol, add 25g of silica gel (J.T. Baker, 60-200 mesh) and stir at 20 to 25°C for 4 days, filter and wash the solids with 100 ml of 10 percent methanol in chloroform. Combine the filtrate and wash and evaporate to a residue under high vacuum. Chromatograph the residue over 300g of alumina (type IV, Woelm) Eluting with 500 ml each of 0.5 percent methanol in chloroform, 1 percent methanol in chloroform, and 5.0 percent methanol in chloroform to obtain the title compound.

Physical data of representative compounds of the invention, obtained by the processes described above, are listed below.

The abbreviations used in this listing are those generally used in the art:

PMR: proton magnetic resonance

MS: mass spectroscopy

CMR: carbon 13 magnetic resonance

$\delta$: parts per million from tetramethyl silane, the internal standard

d: doublet

dd: doublet of a doublet

s: singlet

t: triplet

AB quartet: 4 peaks

J: coupling constant

$CDCl_3$: deuterated chloroform

$CD_3OD$: deuterated methanol

1. 2',4"-DIACETYL ANTIBIOTIC AB5-2.

$C_{41}H_{65}NO_{14}$ (MW 811)

| PMR(Acetone-$d_6$) | MS |
|---|---|
| $H_2$ 6.03δ (d,J=16HZ) | 811 (m+,0.84%) |
| $H_3$ 6.60δ (dd,J=16,10HZ) | 595 (m+-2'-acetyldesosamine,1.14%) |
| $H_{10}$ 6.93δ (d,J=16HZ) | 578 (m+-4"-acetylmycinose, 0.39%) |
| $H_{11}$ 6,30δ (dd,J=16,8HZ) | 217 (2'-acetyldesosamine, 19.5%) |
| $H_{15}$ 5.38δ (dd,J=7,6HZ) | 200 (2'-acetyldesosaminyl, 100%) |
| $H_{1'}$ 4.50δ (d,J=7HZ) | |
| $H_{2'}$ 4.75δ (d,J=10,7HZ) | |
| $H_{1''}$ 4.65δ (d,J=8HZ) | |
| $H_{4''}$ 4.45δ (dd,J=10,2.5HZ) | |
| $N(CH_3)_2$ 2.22δ (s) | |
| 2x $OCH_3$ 3.48δ(s), 3.52δ(s) | |
| 2x OAC 2.05δ(s) | |

2. 2',4"-DIACETYL-12,13-DESEPOXY-12,13-DEHYDRO ANTIBIOTIC AR5-2

$C_{41}H_{65}NO_{13}$ (MW 795)

| PMR($CDCl_3$) | MS |
|---|---|
| $H_2$ 5.78 δ (d,J=16 HZ) | 795 (m+, 2.32 %) |
| $H_3$ 7.14 δ (dd,J=11,15 HZ) | 217 (4"-acetylmycinosyl, 21.48 %) |
| $H_{10}$,$H_{11}$,$H_{12}$,$H_{13}$ (5.85 δ ~ 6.8 δ (m) | 200 (2'-acetyldesosaminyl, 100 %) |
| $H_{15}$ 4.85 δ (m) | |
| $H_{1'}$ 4.30 δ (d,J=7 HZ) | |
| $H_{1"}$ 4.63 δ (d,J=8 HZ) | |
| $H_{2'}$ 4.80 δ (m) | |
| $H_{4"}$ 4.45 δ (dd,J=2.0,10HZ) | |
| 2x $OCH_3$ 3.52 δ (s), 3.48 δ (s) | |
| $N(CH_3)_2$ 2.26 δ (s) | |
| 2x OAC 2.10 δ (s), 2.08 δ (s) | |

# 0033433



3. 11-(P-METHOXYBENZYLTHIO)-10,11-DIHYDRO ANTIBIOTIC AR5-2

$C_{45}H_{71}NSO_{14}$ (MS 881)

<u>PMR(Acetone-$d_6$)</u>

$H_2$ 6.10δ (d,J=16HZ)

$H_3$ 6.78δ (dd,J=16,10HZ)

$H_{15}$ 5.49δ (dd,J=8.0, 4.5HZ)

$H_{1'}$ 4.33δ (d,J=7HZ)

$H_{1''}$ 4.61δ (d,J=8HZ)

$N(CH_3)_2$ 2.28δ (s)

2x $OCH_3$ 3.57δ (s)

1x $OCH_3$ 3.78δ (s, on benzene ring)

2x $H_{2'''}$ 7.27δ (d,J=8.5HZ)

2x $H_3$" 6.87δ (d,J=8.5HZ)

<u>MS</u>

727 (m+-$HSCH_2$ 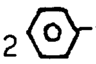 -$OCH_3$, 0.47%)

175 (mycinosyl, 7.24%)

174 (desosamine, 24.98%)

158 (desosaminyl,100%)

122 ($CH_3O$- 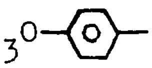 -$CH_2$+H•, 11.69%)

121 ($CH_3O$- 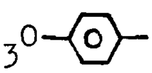 -$CH_2$+̇, 99.81%)

4. 11-ETHYLTHIO-10,11-DIHYDRO ANTIBIOTIC AR5-2

$C_{39}H_{67}NSO_{13}$ (MW 789)

PMR (Acetone-$d_6$)

$H_2$ 6.11δ (d,J=16HZ)

$H_3$ 6.79δ (dd,J=16,10HZ)

$H_{15}$ 5.50δ (dd,J=8.5, 4.5HZ)

$H_{1'}$ 4.34δ (d,J=7HZ)

$H_{1''}$ 4.60δ (d,J=8HZ)

$NMe_2$ 2.31δ (s)

2x $OCH_3$ 3.56 (s)

CMR($CDCl_3$)

$C_1$ 166.126

$C_2$ 120.178

$C_3$ 152.578

$C_9$ 211.872

$C_{10}$ 26.655

$C_{11}$ 42.214

$\underline{CH_2}S$ 37.514

$\underline{CH_3}CH_2S$ 14.891

MS

789 (m+)

728 (m+-$CH_3CH_2S$, 1.84%)

175 (mycinosyl, 5.1%)

174 (desosamine, 18,94%)

158 (desosaminyl, 100%)

5. 9-DIHYDRO-12,13-DESEPOXY-12,13-DEHYDRO ANTIBIOTIC AR5-2

$C_{37}H_{63}NO_{12}$ (MW 713)

PMR(Acetone-$d_6$)

$H_2$,$H_3$,$H_{10}$,$H_{11}$,$H_{12}$,$H_{13}$,5.4~7.10δ (m)

$H_{15}$ 5.10δ (t,J=7.5HZ)

$H_{1'}$ 4.35δ (d,J=7HZ)

$H_{1''}$ 4.55δ (d,J=8HZ)

$NMe_2$ 2.30δ (s)

2x $OCH_3$ 3.52δ (s), 3.55δ(s)

CMR($CDCl_3$)

$C_1$ 167.26

$C_2$ 120.67

$C_3$ 151.85

$C_9$ 76.71

$C_{10}$ 128.45

$C_{11}$ 135.24

$C_{12}$ 130.34

$C_{13}$ 133.98

$C_{1'}$ 104.94

$C_{1''}$ 101.85

MS

713 (m+, 1.26%)

695 (m+-$H_2O$,1.29%)

191 (mycinose, 1.01%)

174 (desosamine,31.57%)

158 (desosamisyl,100%)

UV

$\lambda_{max}^{MEOH}$ : 217nm (ε 28,500)

6. 9,2',4"-TRIACETYL-9-DIHYDRO-12,13-DESEPOXY-12,13-DEHYDRO ANTIBIOTIC AR5-2

$C_{43}H_{69}NO_{15}$ (MW 839)

| PMR ($CDCl_3$) | MS |
|---|---|
| olefinic protons 5.6δ~7.0δ (m) | 841 ($m^+$+2, 1.16%) |
| $H_{1''}$ 4.65δ (d,J=8HZ) | 779 ($m^+$-ACOH, 2.7%) |
| $H_{1'}$ 4.34δ (d,J=8HZ) | 563 ($m^+$-ACOH-2'-acetyl-desosamine, 1.58%) |
| $NMe_2$ 2.30δ (s) | 217 (2'-acetyldesosamine, 59.24%) |
| 2x $OCH_3$ 3.52δ (s) | 200 (2'-acetyldesosaminyl, 100%) |
| -OAC 2.02δ (s) | |
| 2x OAC 2.09δ (s) | |

7. 11-ETHYLTHIO-10,11-DIHYDRO ANTIBIOTIC AR5-1

$C_{39}H_{67}NO_{12}S$ (MW 773)

PMR(Acetone-$d_6$)

$H_2$ 6.05δ (d,J=16HZ)

$H_3$ 6.75δ (d,J=16,10HZ)

$H_{15}$ 5.30δ (m)

$H_{1'}$ 4.30δ (d,J=7HZ)

$H_{1''}$ 4.55δ (d,J=8.0HZ)

$NMe_2$ 2.28δ (s)

2x $OCH_3$ 3.50δ (s), 3.52δ (s)

MS

773 ($m^+$, 0.86%)

712 ($m^+$-$CH_3CH_2S$, 2.28%)

191 (mycinose, 2.25%)

174 (desosamine, 32.3%)

158 (desosaminyl, 100%)

CMR($CDCl_3$)

$C_1$ 165.919

$C_2$ 120.416

$C_3$ 152.117

$C_9$ 211.156

$C_{10}$ 26.480

$C_{11}$ 42.131

$\underline{CH_2}S$ 37.638

$\underline{CH_3}CH_2S$ 14.919

8. 4"-METHANESULFONYL-12,13-DESEPOXY-12,13-DEHYDRO ANTIBIOTIC AR5-2

$C_{38}H_{63}NSO_{14}$ (MW 789)

| PMR(Acetone-$d_6$) | MS |
|---|---|
| $H_2$ 5.93δ (d,J=16HZ) | 789 ($m^+$, 2.55%) |
| $H_3$ 7.13δ (dd,J=16,10HZ) | |
| $H_{10}$ 6.21δ (d,J=15HZ) | 174 (desosamine, 38.47%) |
| $H_{11}$, $H_{12}$, $H_{13}$ 6.4δ-6.8δ(m) | 158 (desosaminyl, 100%) |
| $H_{15}$ 5.04δ (dd,J=8.5, 4.5HZ) | |
| $H_{1'}$ 4.31δ (d,J=7HZ) | |
| $H_{1''}$ 4.69δ (d,J=8.0HZ) | |
| $NMe_2$ 2.52δ (s) | |
| 2x $OCH_3$ 3.53δ (s), 3.57δ (s) | |
| $CH_3SO_2$ 3.25δ (s) | |

9. 2'-ACETYL-9-DIHYDRO-12,13-DESEPOXY-12,13-DEHYDRO ANTIBIOTIC AR5-2

$C_{39}H_{65}NO_{13}$ (MW 755)

PMR ($CDCl_3$)

olefinic protons, 5.50δ~7.50δ(m)

$H_{15}$ 4.90δ (m)

$H_{1'}$ 4.34δ (d,J=7HZ)

$H_{1''}$ 4.60δ (d,J=8HZ)

OAC 2.08δ (s), 2.11δ (s)

$NMe_2$ 2.28δ (s)

MS

755($m^+$, 1.64%)

737 ($m^+$-$H_2O$, 2.91%)

347 ($m^+$-mycinose-2'-acetyl-desosamine 2.25%)

200 (2'-acetyldesosaminyl, 100%)

UV

$\lambda_{max}^{MEOH}$ : 216nm (ε30,500)

10. 2'-ACETYL-4"-DEHYDRO ANTIBIOTIC AR5-2

PMR(Acetone-$d_6$)

$H_2$ 6.1δ (d,J=16HZ)

$H_3$ 6.64δ (dd,J=16,10HZ)

$H_{11}$ 6.35δ (dd,J=16,9HZ)

$H_{10}$ 6.94δ (d,J=16HZ)

$H_{15}$ 5.42δ (m)

$H_{1'}$ 4.45δ (d,J=8HZ)

$H_{1''}$ 4.82δ (d,J=7HZ)

OAC 2.00δ (s)

$N(CH_3)_2$ 2.50δ (s)

2x $OCH_3$ 3.50δ (s), 3.60δ(s)

CMR($CDCl_3$)

$C_1$ 165.89

$C_2$ 120.10

$C_3$ 151.75

$C_9$ 200.87

$C_{10}$ 126.53

$C_{11}$ 142.72

$C_{22}$ 169.87

MS

767 ($m^+$,0.45%)

735 ($m^+$-MEOH, 0.59%)

595 ($m^+$-4"-dehydro-mycinosyl, 3.05%)

216 (2'-acetyldesosamine, 11.24%)

200 (2'-acetyldesosaminyl, 100%)

UV

$\lambda_{max}^{MEOH}$ 214nm ( 24.300)

242nm (ε11,200)

11. 11-ACETAMIDO-10,11-DIHYDRO ANTIBIOTIC AR5-2

$C_{39}H_{66}N_2O_{14}$ (MW 786)

PMR(Acetone-$d_6$ /$CDCl_3$)

$H_2$ 5.98 δ (d,J=16HZ)

$H_3$ 6.78 δ (dd,J=16,10HZ)

$H_{15}$ 5.36 δ (dd,J=8,5HZ)

$H_{1'}$ 4.32 δ (d,J=7HZ)

$H_{1''}$ 4.58 δ (d,J=8HZ)

-NH 6.95 δ (broad)

AC 1.86 δ (s)

$NMe_2$ 2.35 δ (s)

2x $OCH_3$ 3.55 δ (s), 3.58 δ (s)

CMR($CDCl_3$)

$C_1$ 166.20

$C_2$ 120.52

$C_3$ 152.54

$C_9$ 213.89

$C_{10}$ 39.31

$C_{11}$ 48.365

$C_{1'}$ 105.25

$C_{1''}$ 101.41

$C_{22}$ 169.99

$C_{23}$ 17.74

MS

786 ($m^+$,0.44%)

611 ($m^+$-desosamine,3.27%)

437 ($m^+$-desosamine-mycinosyl, 4.53%)

378 (437-$CH_3CONH_2$ ,1.89%)

175 (desosamine, 11.24%)

158 (desosaminyl, 63%)

UV

$\lambda_{max}^{MEOH}$ :215nm (ε15,000)

12. 11-(1-PIPERIDINYL)-10,11-DIHYDRO ANTIBIOTIC AR5-2

$C_{42}H_{72}N_2O_{13}$ (MW 812)

MS

812 ($m^+$, 0.19%)

728 ($m^+$-•N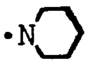, 0.66%)

696 ($m^+$-•N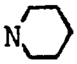$CH_3OH$, 1.08%)

582 ($m^+$-145-HN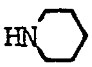2.23%)

452 ($m^+$-mycinosyl-101- N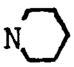 ,10.50%)

361 ($m^+$- N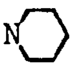 – desosamine-mycinose, 5.29%)

175 (mycinosyl, 22.68%)

174 (desosamine, 66%)

158 (desosaminyl, 99.83%)

UV

$\lambda_{max}^{MEOH}$ :214nm (ε12.230)

13. 11-(2-AMINO-2-ETHOXYCARBONYLETHANETHIO) 10,11-DIHYDRO ANTIBIOTIC AR-5-2

$C_{42}H_{72}N_2SO_{15}$ (NW 876)

MS

```
                   NH2
                   |
728  (m+-EtOC-C-CH2S , 0.20%)
             ||  H
             O
                     NH2
                     |
452  (m+-EtO-C-C-CH2SH-mycinosyl-101,1.81%)
              ||  H
              O
```

202 (17.25%)

175 (mycinosyl, 6.85%)

174 (desosamine, 37.42%)

158 (desosaminyl, 45.41%)

102 (100%)

14. 11-MORPHOLINO-10,11-DIHYDRO ANTIBIOTIC AR5-2

$C_{41}H_{70}N_2O_{14}$ (814)

<u>MS</u>

814 ($M^+$, 0.15%)

728 ($m^+$- • 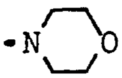 1.07%

696 ($m^+$- • 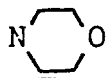 $CH_3OH$, 2.28%)

582 ($m^+$-HN 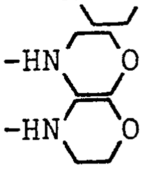 145, 3.67%)

452 ($m^+$-HN O mycinosyl-101, 9.19%)

175 (mycinosyl, 26.17%)

174 (desosamine, 96.7%)

158 (desosaminyl, 100%)

<u>UV</u>

$\lambda_{max}^{MEOH}$ : 214 nm($\epsilon$13.800)

15. 11-(4-METHYLPIPERAZINYL)-10,11-DIHYDRO ANTIBIOTIC AR5-2

$C_{42}H_{73}N_3O_{13}$ (MW 827)

MS

827 ($m^+$, 0.18%)

728 ($m^+$ - 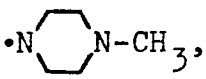, 0.34%)

653 ($m^+$-desosamine, 0.6%)

363 ($m^+$-desosamine-mycinose, 1.39%)

175 (mycinosyl, 14.84%)

174 (desosamine) 58%)

158 (desosaminyl, 100%)

UV

$\lambda_{max}^{MEOH}$ : 214nm ($\epsilon$ 14.242)

16. 11-THIOMORPHOLINO-10,11-DIHYDRO ANTIBIOTIC AR5-2

$C_{41}H_{70}N_2SO_{13}$ (MW 830)

MS

728 ($m^+$-• 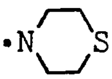, 0.23%)

452 ($m^+$- 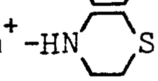 mycinosyl-101, 3.35%)

362 ($m^+$- 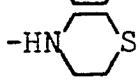 mycinose-desosamine, 2.58%)

175 (mycinosyl, 10.94%)

174 (desosamine, 26.08%)

158 (desosaminyl, 91.07%)

UV

$\lambda_{max}^{MEOH}$ : 209nm ($\epsilon$14,715)

212nm ($\epsilon$14,470)

17. 10,11-DIHYDRO ANTIBIOTIC AR5-1

$C_{37}H_{63}NO_{12}$ (MW 713)

PMR(Acetone-$d_6$)

$H_2$ 6.11δ (d,J=15HZ)

$H_3$ 6.86δ (dd,J=15.10HZ)

$H_{15}$ 5.2~5.5δ (m)

$H_{1'}$ 4.36δ (d,J=8HZ)

$H_{1''}$ 4.56δ (d,J=8HZ)

$NMe_2$ 2.30δ (s)

2x $OCH_3$ 3.50δ(s), 3.54δ(s)

MS

713 ($m^+$, 2.29%)

568 ($m^+$-145, 1.73%)

522 ($m^+$-mycinose,2.44%)

347 ($m^+$-mycinose-desosamine, 3.13%)

195 (mycinosyl, 5.23%)

174 (desosamine, 18.68%)

158 (desosaminyl, 100%)

CMR($CDCl_3$)

$C_9$ 213.46

$C_{10}$ 32.97

$C_{11}$ 26.50

$C_1$ 166.09

$C_2$ 120.71

$C_3$ 151.92

UV

$\lambda_{max}^{MEOH}$ : 214nm (ε15.700)

277nm (ε2.010)

18. 4"-ACETYL-12,13-DESEPOXY-12,13-DEHYDRO ANTIBIOTIC AR5-2

$C_{39}H_{63}NO_{13}$ (MW 753)

PMR(Acetone-$d_6$)

$H_2$ 5.85δ (d,J=16HZ)

$H_3$ 7.10δ (dd,J=16, 10HZ)

$H_{10}$ 6.20δ (d,J=15HZ)

$H_{11}$, $H_{12}$, $H_{13}$, 6.3~6.8δ(m)

$H_{15}$ 5.0δ (dd,J=7.5, 7HZ)

$H_{1'}$ 4.25δ (d,J=7HZ)

$H_{1''}$ 4.60δ (d,J=8.HZ)

$H_{4''}$ 4.4δ (dd,J=10, 2.5HZ)

$NMe_2$ 2.25δ (s)

-OAC 2.05δ (s)

2x $OCH_3$ 3.50δ (s)

MS

753 ($m^+$, 3.94%)

217 (4"-acetylmycinosyl 64%)

174 (desosamine, 20.82%)

158 (desosaminyl 100%)

UV

$\lambda_{max}^{MEOH}$ : 225nm ( 18.000)

278nm ( 21.100)

Rotation

$[\alpha]_D^{26}$: +74.2°

19. 4"-ACETYL ANTIBIOTIC AR5-2

$C_{39}H_{63}NO_{14}$ (MW 769)

PMR(Acetone-$d_6$)

$H_2$ 6.03δ(d,J=16HZ)

$H_3$ 6.60δ(dd,J=16.10HZ)

$H_{10}$ 6.90δ(d,J=16HZ)

$H_{11}$ 6.30δ(dd,J=16.8HZ)

$H_{15}$ 5.4 δ(dd,J=7.6HZ)

$H_{1'}$ 4.25δ(d,J=7HZ)

$H_{1''}$ 4.65δ(d,J=8HZ)

$H_{4''}$ 4δ (dd,J=10, 2HZ)

$NMe_2$ 2.26δ(s)

-OAC 2.04δ(s)

2x $OCH_3$ 3.49δ(s), 3.53δ(s)

MS

769 ($m^+$, 0.36%)

217 (4"-acetylmycinosyl, 20.7%)

174 (desosamine, 15.94%)

158 (desosaminyl, 100%)

20. 9-DIHYDRO-12,13-DESEPOXY-12,13-DEHYDRO ANTIBIOTIC AR5-1

$C_{37}H_{65}NO_{12}$ (MW 715)

PMR(Acetone-$d_6$)

$H_2$, $H_3$, $H_{12}$, $H_{13}$, 5.50δ~6.98(m)

$H_{15}$ 5.12δ (t,J=7HZ)

$H_{1'}$ 4.36δ (d,J=7HZ)

$H_{1''}$ 4.60δ (d,J=8HZ)

$N(CH_3)_2$ 2.30δ (s)

2x $OCH_3$ 3.56δ(s), 3.54δ(s)

MS

715 ($m^+$, 1.83%)

175 (mycinosyl, 11.46%)

174 (desosamine, 30.88%)

158 (desosaminyl, 100%)

349 ($m^+$-desosamine-mycinose, 1.57%)

UV

$\lambda_{max}^{MEOH}$ : 216nm (ε 24.600)

21. 9-DIHYDRO ANTIBIOTIC AR5-2

$C_{37}H_{63}NO_{13}$ (MW 729)

PMR(Acetone-$d_6$)

$H_2$ 5.70δ (d,J=16HZ)

$H_3$ 6.81δ (dd,J=16,10HZ)

$H_{15}$ 5.40δ (m)

$H_{1'}$ 4.35δ (d,J=7HZ)

$H_{1''}$ 4.58δ (d,J=8HZ)

$NMe_2$ 2.28δ (s)

2x $OCH_3$ 3.56δ (s)

MS

729 ($m^+$, o.54%)

711 ($m^+$-$H_2O$, 0.39%)

175 (mycinosyl,6.33%)

174 (desosamine, 20.64%)

158 (desosaminyl, 100%)

UV

$\lambda_{max}^{MEOH}$ :214nm (ε16,200)

22. 2'-ACETYL ANTIBIOTIC AR5-2

$C_{39}H_{63}NO_{14}$ (MW 769)

PMR(Acetone-$d_6$)

$H_2$ 6.05 δ (d,J=16HZ)

$H_3$ 6.58 δ (dd,J=16.10HZ)

$H_{10}$ 6.92 δ (d,J=16HZ)

$H_{11}$ 6.30 δ (dd,J=16.8HZ)

$H_{15}$ 5.38 δ (dd,J=7, 5.5HZ)

$H_{1'}$ 4.5 δ (d,J=7HZ)

$H_{1''}$ 4.55 δ (d,J=8HZ)

$H_{2'}$ 4.82 δ (dd,J=10.7HZ)

$NMe_2$ 2.46 δ (s)

-OAC 2.11 δ (s)

2x $OCH_3$ 3.54 δ (s)

MS

769 ($m^+$)

361 ($m^+$-2'-acetyl-desosamine-mycinose,1.56%)

200 (2'-acetyl-desosaminyl, 100%)

23. 9-DIHYDRO ANTIBIOTIC AR5-1

$C_{37}H_{63}NO_{12}$ (MW 713)

PMR(Acetone-$d_6$)

$H_2$ 5.90δ (d,J=16HZ)

$H_3$ 6.85δ (dd,J=16,10HZ)

$H_{15}$ 5.30δ (m)

$H_{1'}$ 4.33δ (d,J=7HZ)

$H_{1''}$ 4.55δ (d,J=8HZ)

$NMe_2$ 2.25δ (s)

2x $OCH_3$ 3.52δ (s), 3.55δ (s)

MS

713 ($m^+$, 0.26%)

347 ($m^+$-desosamine-mycinosyl, 1.07%)

174 (desosamine, 23.67%)

158 (desosaminyl, 100%)

UV

$\lambda_{max}^{MEOH}$ : 214nm (ε 17,060)

24. DESMYCINOSYL-12,13-DESEPOXY-12,13-DEHYDRO ANTIBIOTIC AR5-2.

$C_{29}H_{47}NO_8$ (MW 537)

PMR($CDCl_3$+$CD_3OD$)

| | |
|---|---|
| $H_2$ | 5.86δ (d,J=16HZ) |
| $H_3$ | 7.18δ (dd,J=16,10HZ) |
| $H_{10}$,$H_{11}$,$H_{12}$,$H_{13}$ | 6.0~6.8δ (m) |
| $H_{15}$ | 4.93δ (dd,J=9.4HZ) |
| $H_{21}$ | 3.61δ (AB quartet,J=11HZ) |
| $H_{1'}$ | 4.26δ (d,J=8HZ) |
| $NMe_2$ | 2.32δ (s) |

MS

537 ($m^+$, 0.92%)

436 ($m^+$-101, 1.03%)

240 ($m^+$-297, 1.38%)

174 (desosamine, 14%)

158 (desosaminyl,100%)

UV

$\lambda_{max}^{MEOH}$ : 214nm (ε22,000)
298nm (ε21,400)

CMR($CDCl_3$+$CD_3OD$)

| | | | |
|---|---|---|---|
| $C_1$ | 167.950 | $C_{2'}$ | 71.622 |
| $C_2$ | 121.745 | $C_{3'}$ | 65.666 |
| $C_3$ | 152.886 | $C_{4'}$ | 30.931 |
| $C_4$ | 41.846 | $C_{5'}$ | 69.867 |
| $C_5$ | 87.884 | $C_{6'}$ | 21.242 |
| $C_6$ | 34.988 | $NMe_2$ | 40.706 |
| $C_7$ | 33.384 | | |
| $C_8$ | 45.671 | | |
| $C_9$ | 206.294 | | |
| $C_{10}$ | 124.660 | | |
| $C_{11}$ | 145.115 | | |
| $C_{12}$ | 130.902 | | |
| $C_{13}$ | 143.139 | | |
| $C_{14}$ | 78.628 | | |
| $C_{15}$ | 77.196 | | |
| $C_{16}$ | 21.325 | | |
| $C_{17}$ | 10.758 | | |
| $C_{18}$ | 19.867 | | |
| $C_{19}$ | 17.909 | | |
| $C_{20}$ | 17.732 | | |
| $C_{21}$ | 65.754 | | |
| $C_{1'}$ | 105.593 | | |

Claims for Austria

1. Process for the preparation of macrolide antibiotics of the general formula I,

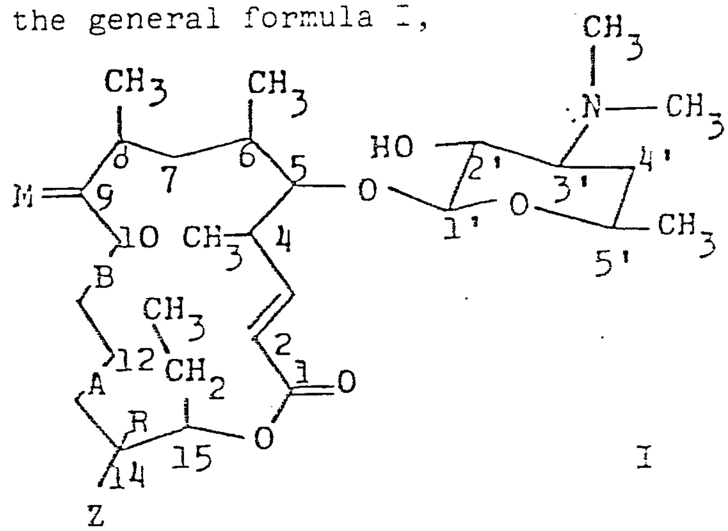

I

wherein R is H or OH; M is O or (H,OH); Z is the group of formula II

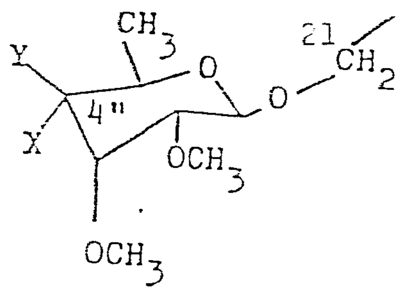

II

wherein X is hydrogen, OH, mesyloxy, tosyloxy, trifluoromethanesulfonyloxy, halo, azido, amino, isocyanato, isothiocyanato, guanidino, $-NHR^6$, $-NR^6R^7$, $-NHR^8$, $-NHC(O)OR^6$, $-NHC(O)OR^8$, $-NHC(NH)R^6$, $-NH(CH_2)pOH$ or $-NH(CH_2)pOR^{10}$, wherein p is an integer of from 2 to 18, or $-NHCOR^6$ when Y is hydrogen; or Y in combination with X is oxo, $=N-NH_2$, $=N-NHR^{11}$ or $=NR^{11}$;

A represents a double bond between the carbon atoms of positions 12 and 13 or, together with the carbon

atoms of positions 12 and 13, an oxirane ring; B represents a double bond between the carbon atoms of positions 10 and 11 or, provided R is H, M is O, X is OH, Y is H, and A together with the carbon atoms of positions 12 and 13 represents an oxirane ring, B may also be a single bond between the carbon atoms of positions 10 and 11, or, provided A together with the carbon atoms of positions 12 and 13 represents an oxirane ring and X is OH and Y is H, B together with the carbon atoms of positions 10 and 11 may also represent the group $-CH_2-CH(Q^1)-$ or $-CH_2-CH(Q^2)-$, wherein $Q^1$ is a nitrogen containing moiety and $Q^2$ is a sulfur containing moiety (the nitrogen or sulfur respectively being attached to the carbon atom of position 11), wherein $-Q^1$ is $-NH_2$, $-NHC(O)R^6$, $-NHR^6$, $-NR^6R^7$, $-NHR^{10}OH$, $-N=CHR^6$, $-NHC(S)NHR^6$, $-NHC(NH)R^6$, $-NHC(O)OR^6$, $-NHC(O)NHR^6$, $-NHC(NH)NH_2$,

-NH-C(O)- 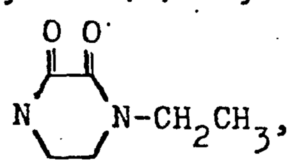, $-NHC(NH)NHR^6$, an amino acid residue, an esterified amino acid residue or a heterocyclic radical of 3 to 7 members containing either nitrogen as the only heteroatom or nitrogen and a second heteroatom which is nitrogen, oxygen, or sulfur;

$-Q^2$ is $-S-R^6$, thioxanthyl, $-S-R^{10}-NHR^6$, $-S-R^8$, $S-R^9$, $-S-R^{10}-R^9$, $-S-R^{10}-R^8-R^9$, $-S-R^8-R^9$, $-S-R^{10}-C(O)OR^6$, $-S-R^{10}-COOH$, a sulfur containing amino acid residue, or a sulfur containing esterified amino acid residue;

and wherein, provided A and B represent double bonds, Z may also be $-CH_2OH$, $-CHO$, $-CH_2NH_2$, $-CH_2NHR^2$, $-CH_2NR^2R^3$, $-CH_2OCOR^2$, $-CH_2NHCOR^2$, $-CH_2NR^3COR^2$, $-CH_2NH-C(=NH)-R^2$, $-CH_2NHC(=NH)-NHR^5$, $-CH_2NHCO-$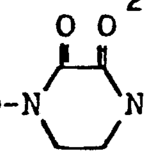$-CH_2CH_3$, $-CH_2O$-glycosyl excluding $-CH_2O$-mycinosyl and $-CH_2O$-(3"-desmethyl-mycinosyl), $-CH_2$-ureido or $CH_2$-thioureido,

wherein $R^2$ and $R^3$ independent of each other represent an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen, or in the amino groups one of substituents $R^2$ and $R^3$ is hydrogen and the other is the group $-(CH_2)_mOR^4$ or 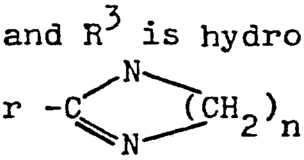, wherein m is an integer of from two to ten, n is an integer of from 2 to 6 and $R^4$ is an alkyl group containing 1 to 18 carbon atoms, an aralkyl group containing 7 to 18 carbon atoms or a heterocyclic group containing 3 to 7 carbon atoms and one hetero atom which is sulfur, nitrogen or oxygen; and $R^5$ is hydrogen, $R^2$ or $-CH_2CH_2-$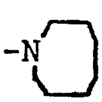 ; $R^6$ and $R^7$ independent of each other represent an alkyl group containing 1 to 18 carbon atoms, an aralkylgroup containing 7 to 18 carbon atoms; $R^8$ is an aryl group; $R^9$ is a heterocyclic group which contains 3 to 7 members, wherein the hetero atoms(s)

is (are) selected from sulfur, nitrogen and oxygen; and $R^{10}$ is an alkyl group containing 1 to 18 carbon atoms; $R^{11}$ represents an alkyl group containing 1 to 18 carbon atoms, an aralkyl group containing 7 to 18 carbon atoms or a heterocyclic group containing 3 to 7 members wherein the hetero atom(s) is(are) selected from sulfur, nitrogen and oxygen; and the non-toxic pharmaceutically acceptable esters thereof and the non-toxic acid addition salts of the compounds of formula I and of their esters, in particular compounds of formula I in their free form or in the form of an ester, wherein the hydroxy group at position 9 and/or 21 and/or 2' and/or 4" is/are esterified with mono-carboxylic acid(s) or di-carboxylic acid(s) each having 2 to 18 carbon atoms, preferably wherein the hydroxy group(s) is(are) esterified with a mono-carboxylic acid containing 2 or 3 carbon atoms; characterized in that the compounds are prepared by an appropriate process or combination of processes selected from the following processes:

-) for the preparation of compounds of the general formula XIV

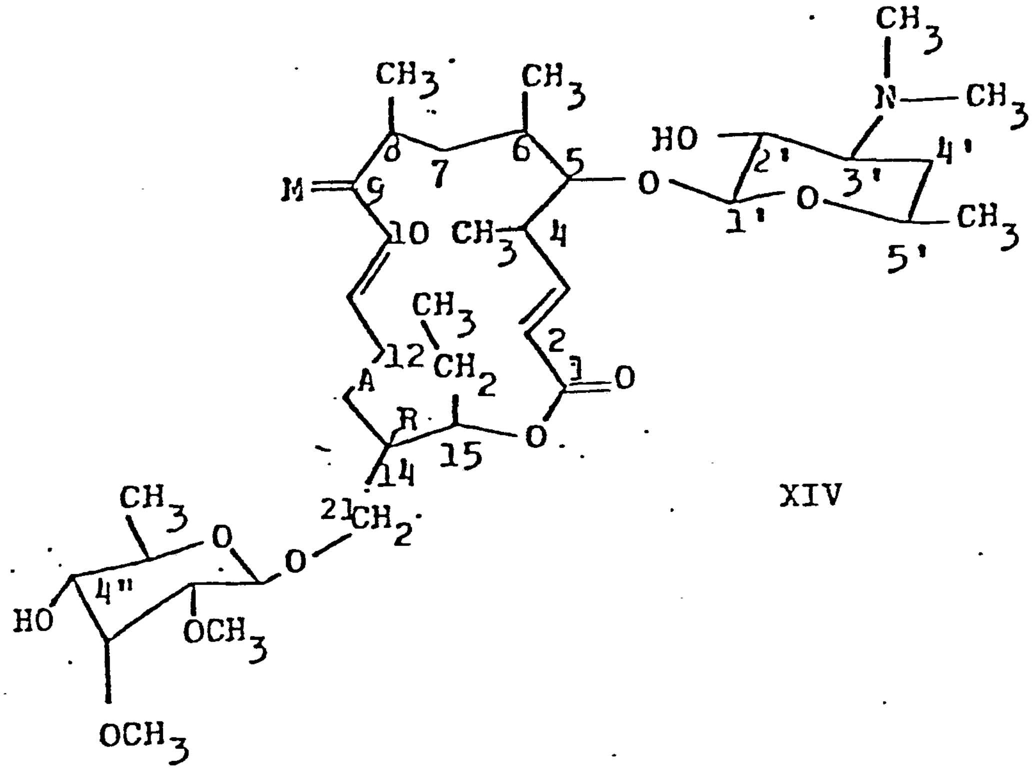

XIV

wherein R is H or OH, M is oxo, and A represents a double bond between the carbon atoms of positions 12 and 13 or, together with the carbon atoms of positions 12 and 13, an oxirane ring: incubation of a microorganism of the species Micromonospora polytrota in an aqueous nutrient medium, under aerobic conditions and isolation of at least one of the compounds of formula XIV therefrom in the free form or in the form of a pharmaceutically acceptable ester or salt;

-) for the preparation of compounds of formula I or their esters, wherein A represents a double bond between the carbon atoms of positions 12 and 13: reduction of a corresponding compound of formula I or its ester wherein A together with the carbon atoms of positions 12 and 13 represents an oxirane ring;

-) for the preparation of 10,11-dihydro antibiotic AR5-1: enzymatic reduction of antibiotic AR5-1;

-) for the preparation of compounds of the general formula I, wherein R is hydrogen or hydroxy, M is oxo, A and B represent double bonds between the carbon atoms of positions 12 and 13, and 10 and 11 respectively, and Z is $-CH_2OH$: hydrolysis of compounds of formula I or their esters, wherein R, M, A and B are defined as above and Z is $-CH_2O$-glycosyl;

-) for the preparation of compounds of the general formula I or their esters, wherein M is oxo or (H,OH), R is hydrogen or hydroxy, A and B represent double bonds between the carbon atoms of positions 12 and 13, and 10 and 11, respectively, and Z is -CHO, $-CH_2NH_2$, $-CH_2NHR^2$, $-CH_2NR^2R^3$, $-CH_2OR^2$, $-CH_2OCOR^2$, $-CH_2NHCOR^2$, $-CH_2NR^3COR^2$, $-CH_2NH-$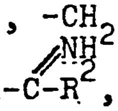, $-CH_2NH$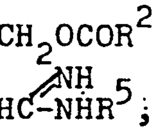;

$-CH_2NHCO-$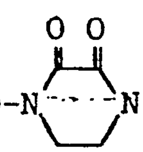$-CH_2CH_3$, $-CH_2O$-glycosyl excluding $-CH_2O$-mycinosyl and $-CH_2O$-(3"-desmethyl-mycinosyl), $-CH_2$-ureido or $-CH_2$—thioureido, wherein $R^2$, $R^3$ and $R^5$ are as defined above:

transformation of the corresponding compound of formula I or its ester, wherein Z is $-CH_2OH$;

-) for the preparation of compounds of the general formula I or their esters, wherein R is hydrogen or hydroxy, M is oxo, A and B represent double bonds between the carbon atoms of positions 12 and 13, and 10 and 11, respectively, and Z is -CHO: oxidation of the corresponding compound of formula I or its ester;

-) for the preparation of compounds of the general formula I or their esters, wherein R is hydrogen or hydroxy, M is oxo or (H,OH), A and B represent double bonds between the carbon atoms of positions 12 and 13, and 10 and 11, respectively, and Z is $-CH_2O$-glycosyl excluding $-CH_2O$-mycinosyl and $-CH_2O$-(3"-desmethyl-mycinosyl): reaction of the corresponding compound of formula I, wherein Z is $-CH_2OH$ or a reactive derivative thereof with the appropriate sugar in the presence of a catalyst and in the absence of water;

-) for the preparation of compounds of the general formula I or their esters, wherein R is hydrogen or hydroxy, M is oxo or (H,OH), A and B represent double bonds between the carbon atoms of positions 12 and 13, and 10 and 11, respectively, and Z is $-CH_2OR^2$, $R^2$ being

BAD ORIGINAL

defined as above: etherification of the corresponding compound of formula I wherein Z is $-CH_2OH$, and wherein, if desired, amino groups and hydroxy groups have been protected;

-) for the preparation of compounds of the general formula I or their esters, wherein R is hydrogen or hydroxy, M is oxo or (H,OH), A and B represent double bonds between the carbon atoms of positions 12 and 13, and 10 and 11, respectively, and Z is $-CH_2NH_2$, $-CH_2NHR^2$ or $-CH_2NR^2R^3$ ($R^2$ and $R^3$ being defined as above): reductive amination of the corresponding compound of formula I or its ester, wherein Z is -CHO; followed by oxidation, if in the desired product M is oxo;

-) for the preparation of compounds of the general formula I or their esters, wherein R is hydrogen or hydroxy, M is oxo or (H,OH), A and B represent double bonds between the carbon atoms of positions 12 and 13, and 10 and 11, respectively, and Z is

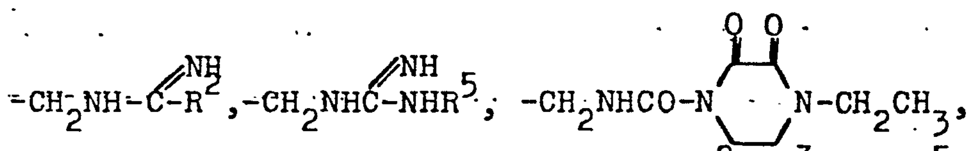, $-CH_2$-ureido or $-CH_2$—thioureido, wherein $R^2$, $R^3$ and $R^5$ are defined as above:

reaction of the corresponding compound of formula I or its 2'-ester, wherein Z is $-CH_2NH_2$ with a compound of

BAD ORIGINAL

formula (V) L-Q, wherein Q is the moiety to be introduced in the primary amino group at position 21 of the starting material and L is a leaving group;

-) for the preparation of compounds of the general formula I or their esters, wherein R is hydrogen or hydroxy, M is oxo or (H,OH), A and B represent double bonds between the carbon atoms of positions 12 and 13, and 10 and 11, respectively, and Z is $-CH_2NHCOR^2$ or $-CH_2NR^3COR^2$:
acylation of the corresponding compound of formula I wherein Z is $-CH_2NH_2$ or $-CH_2NHR^2$;

-) for the preparation of compounds of formula I, wherein M is oxo, and A, B, R and Z are as defined above except compounds wherein Z is $-CH_2OH$ or X is OH:
oxidation of the corresponding compound of formula I, wherein M is (H,OH);

-) for the preparation of compounds of formula I wherein M is (H,OH) and A, B, R and Z are as defined above, with the proviso that Z is other than -CHO and the proviso that, if Z is the group of formula II Y is hydrogen:
reduction of the corresponding compound of formula I, wherein M is O or, if in the desired product Z is

BAD ORIGINAL

$-CH_2OH$ or the group of formula II, wherein X is hydroxy, the starting compound may also be a compound of formula I, wherein Z is -CHO or Y in combination with X is oxo;

-) for the preparation of compounds of the general formula VIII or IX or their esters,

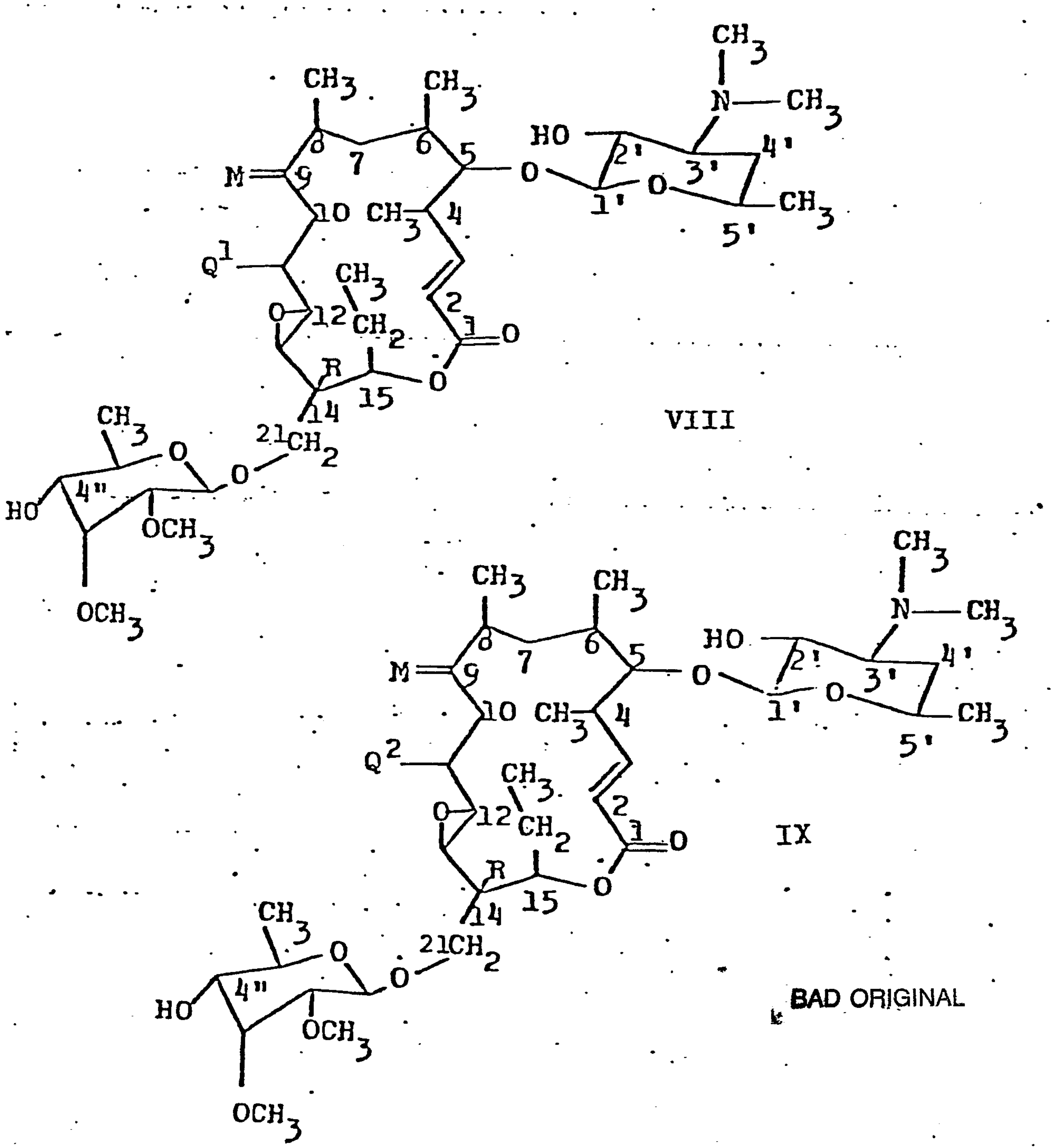

VIII

IX

BAD ORIGINAL

wherein R is H or OH, M is O, $Q^1$ is $-NH_2$, $-NHR^6$, $-NR^6R^7$, $-NHC(NH)NH_2$, $-NHC(NH)NHR^6$, an amino acid residue, an esterified amino acid residue or a heterocyclic radical of 3 to 7 members containing either nitrogen as the only heteroatom or nitrogen and a second heteroatom which is nitrogen, oxygen, or sulfur; and $Q^2$ is $-S-R^6$, thioxanthyl, $-S-R^{10}-NHR^6$, $-S-R^8$, $S-R^9$, $-S-R^{10}-R^9$, $S-R^{10}-R^8-R^9$, $-S-R^8-R^9$, $-S-R^{10}-C(O)OR^6$, $-S-R^{10}-COOH$, a sulfur containing amino acid residue, or a sulfur containing esterified amino acid residue; and $R^6$, $R^7$, $R^8$, $R^9$ and $R^{10}$ are as defined for formula I above:

reaction of antibiotic AR5-1, antibiotic AR5-2 or the esters of these compounds with an appropriate compound of the formula $HQ^1$(VI) or $HQ^2$ (VII) respectively, or with a reactive derivative of the compound of formula VI or VII;

-) for the preparation of compounds of the general formula VIII, wherein $Q^1$ is $-NH-C(O)NHR^6$ or $-NHC(S)NHR^6$, R is H or OH and M is O or (H,OH) and $R^6$ is as defined for formula I above: reaction of the corresponding compound of formula VIII, wherein $Q^1$ is amino with an isocyanate or isothiocyanate of the general formula $R^6NCO$ (XI) or $R^6NCS$ (XII), respectively;

BAD ORIGINAL

-) for the preparation of compounds of the general formula VIII, wherein $Q^1$ is $-NHR^6$, $-NR^6R^7$ or an esterified amino acid residue $-NH\overset{R^6}{C}H-COOR^7$, R is H or OH and M is O or (H,OH), and $R^6$ and $R^7$ are defined as for formula I above:

reaction of the corresponding compound of formula VIII, wherein $Q^1$ is $-NH_2$ with the appropriate aldehyde ($R^6CHO$), ketone ($R^6COR^7$) or 2-oxo-carboxylic acid ($R^6C(O)COOR^7$) in the presence of a reducing agent such as sodiumcyanoborohydride, sodium borohydride or lithium tri-tertiary butoxy aluminium hydride, followed if desired, by oxidation of the so obtained 9-hydroxy compound to provide the corresponding 9-oxo compound;

-) for the preparation of compounds of the general formula VIII, wherein Q1 is $-NHC(O)R^6$, $-NHC(O)OR^6$,

-NHC(O)-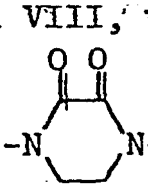-$CH_2CH_3$, or $-N=CHR^6$, R is H or OH and M is O or (H,OH), and $R^6$ is defined as for formula I above:

reaction of the corresponding compound of formula VIII, wherein $Q^1$ is $-NH_2$ with a compound containing, besides a leaving group, the group $-C(O)R^6$, $-C(O)OR^6$

-C(O)-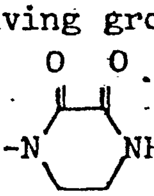$HC_2CH_3$ or $=CHR^6$, respectively;

BAD ORIGINAL

-) for the preparation of compounds of the general formula XIII or their esters,

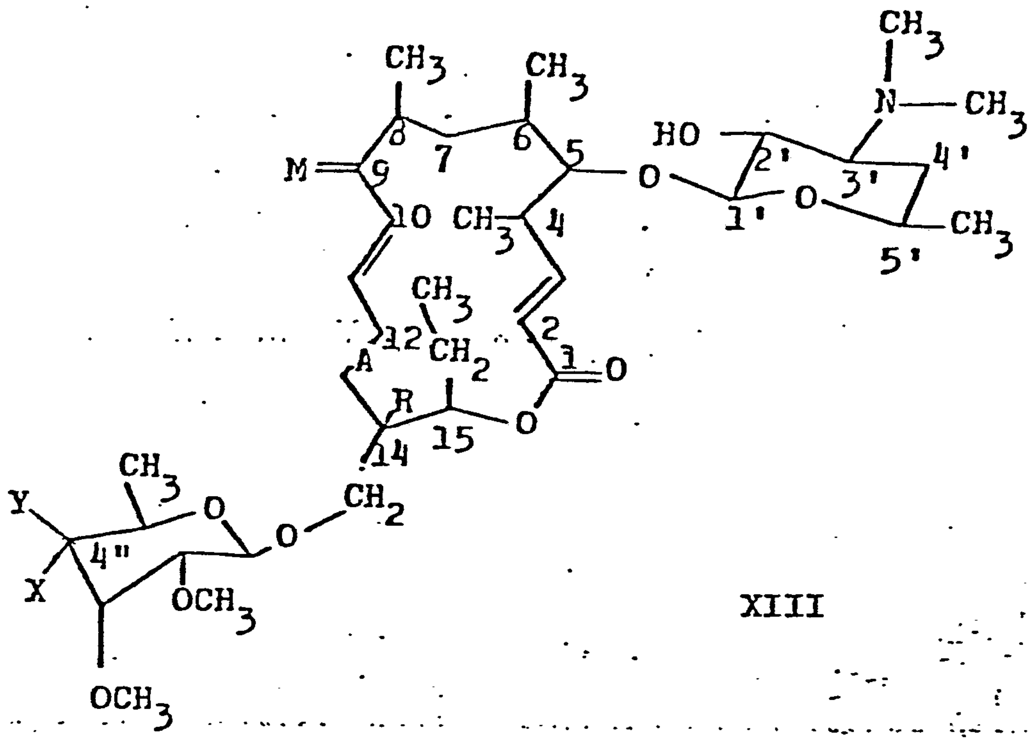

XIII

wherein R is H or OH; M is oxo or (H,OH); A represents a double bond between the carbon atoms of positions 12 and 13 or, together with the carbon atoms of positions 12 and 13, an oxirane ring, and X is mesyloxy: reaction of the corresponding compound of formula XIII or its ester, wherein X is hydroxy and Y is hydrogen with mesylchloride;

-). for the preparation of compounds of the general formula XIII or their esters, wherein R, M and A are as defined above and X is tosyloxy or trifluoromethane sulfonyloxy and Y is hydrogen: reaction of the corresponding compound of formula XIII or its ester, wherein X is hydroxy and Y is hydrogen with tosylchloride or trifluoromethane sulfonylchloride, respectively;

BAD ORIGINAL

-) for the preparation of compounds of the general formula XIII or their esters, wherein R, M and A are as defined above and X together with Y represents oxo: oxidation of the corresponding compound of formula XIII or its ester, wherein X is hydroxy and Y is hydrogen;

-) for the preparation of compounds of the general formula XIII or their esters, wherein R, M and A are as defined above and X is azido and Y is hydrogen: reaction of the corresponding compound of formula XIII or its ester, wherein X is trifluoromethane sulfonyloxy with sodium azide;

-) for the preparation of compounds of the general formula XIII or their esters, wherein R, M and A are as defined above and X halo and Y is hydrogen: reaction of the corresponding compound of formula XIII or its ester, wherein X is trifluoromethane sulfonyloxy with an alkali metal halide;

-) for the preparation of compounds of the general formula XIII or their esters, wherein R, M and A are as defined above and X is hydroxy which has the opposite configuration to that of the hydroxy group of the starting material and Y is hydrogen: reaction of the corresponding triflate with sodium acetate in acetic

acid followed by hydrolysis of the so obtained 4"-acetate;

-) for the preparation of compounds of the general formula XIII or their esters, wherein R, M and A are as defined above and X is O=C=N- or S=C=N- and Y is hydrogen:
reaction of the corresponding compound of formula XIII or its ester, wherein X is azido with triphenylphosphine and carbon dioxide or triphenylphosphine and carbon disulfide, respectively;
-) for the preparation of compounds of the general formula XIII or their esters, wherein R, M and A are as defined above and X is amino and Y is hydrogen:
reaction of the corresponding compound of formula XIII or its ester wherein X is azido with triphenylphosphine;

-) for the preparation of compounds of the general formula XIII or their esters, wherein R, M and A are as defined above and X is amino and Y is hydrogen:
reaction of the ester of the corresponding 4"-halo compound with hexamethylene tetramine;

-) for the preparation of compounds of the general formula XIII or their esters, wherein R, M and A are as defined above and X and Y are hydrogen:

BAD ORIGINAL

dehalogenation of the corresponding 4"-halo compound;

-) for the preparation of compounds of the general formula XIII or their esters, wherein R, M and A are as defined above and X is $-NHR^6$, $-NR^6R^7$ or $-NHR^8$: reaction of the corresponding 4"-amino compound or its ester, with an aldehyde ($R^6CHO$ or $R^8CHO$) or ketone ($R^6COR^7$), respectively, in the presence of a reducing agent such as sodium cyanoborohydride, sodium borohydride or lithium tritertiary butoxy aluminium hydride, in a non-reactive solvent, ($R^6$, $R^7$ and $R^8$ being defined as above for formula I), followed, if desired, by oxidation of the so obtained 9-hydroxy compound to the corresponding 9-oxo compound;

-) for the preparation of compounds of the general formula XIII or their esters, wherein R, M and A are as defined above and X is $-NHC(O)OR^6$ or $-NHC(O)OR^8$ and Y is hydrogen:
reaction of the corresponding 4"-amino compound or its ester with a chloroformate ($ClCOOR^6$ or $ClCOOR^8$) in the presence of sodium carbonate and water ($R^6$ and $R^8$ being defined as above for formula I);

-) for the preparation of compounds of the general

BAD ORIGINAL

formula XIII or their esters, wherein R, M and A are as defined above and X $-NHCOR^6$ ($R^6$ being as defined above)and Y is hydrogen: reaction of the corresponding 4"-amino compound or its ester with the anhydride or halide of the acid $R^6COOH$ in a non reactive solvent;

-) for the preparation of compounds of the general formula XIII or their esters, wherein R, M and A are as defined above and X is guanidino or $-NHC(NH)R^6$ ($R^6$ being as defined above): reaction of the corresponding 4"-amino compound or its ester with S-methyl isothiourea or an imino ether, respectively;

-) for the preparation of compounds of the general formula XIII or their esters, wherein R,M and A are as defined above and X is $-NH(CH_2)pOH$ or $-NH(CH_2)pOR^{10}$, wherein p is an integer of from 2 to 18, $R^{10}$ is as defined above; reaction of the corresponding 4"-amino compound or its ester with hal-$(CH_2)pOH$ or hal-$(CH_2)pOR^{10}$ ($R^{10}$ and p being as defined above, hal representing halogen) in the presence of a base;

-) for the preparation of compounds of the general formula XIII or their esters, wherein R, M and A are as defined above and X is hydroxy and Y is hydrogen: reduction of the corresponding compound of formula XIII or its ester wherein X in combination with Y is oxo, followed if desired by oxidation of the so obtained 9-hydroxy compound to obtain the corresponding 9-oxo

BAD ORIGINAL

compound;

-) for the preparation of compounds of the general formula XIII or their esters, wherein R, M and A are as defined above and X in combination with Y is $=N-NH_2$, $=N-NHR^{11}$ or $=NR^{11}$ ($R^{11}$ being defined as above): reaction of the corresponding compound of formula XIII or its ester, wherein X in combination with Y is oxo, with hydrazine, substituted hydrazine $R^{11}-NH-NH_2$ or a primary amine $R^{11}NH_2$, respectively;

The above described processes being followed, if desired by the isolation of the isomers of the so obtained compounds of formula I and/or transformation of the so obtained compounds of formula I into their esters and/or hydrolysis of the so obtained esters of compounds of formula I into the free form and/or transformation of the so obtained compounds into their acid addition salts.

2. Process according to claim 1, wherein the incubation is performed under submerged conditions in a nutrient medium containing assimilable sources of at least nitrogen and carbon, preferably using <u>Micromonospora polytrota</u> NRRL 12066.

3. A process according to claim 1 or 2, characterized in that a compound of formula XIV is prepared,

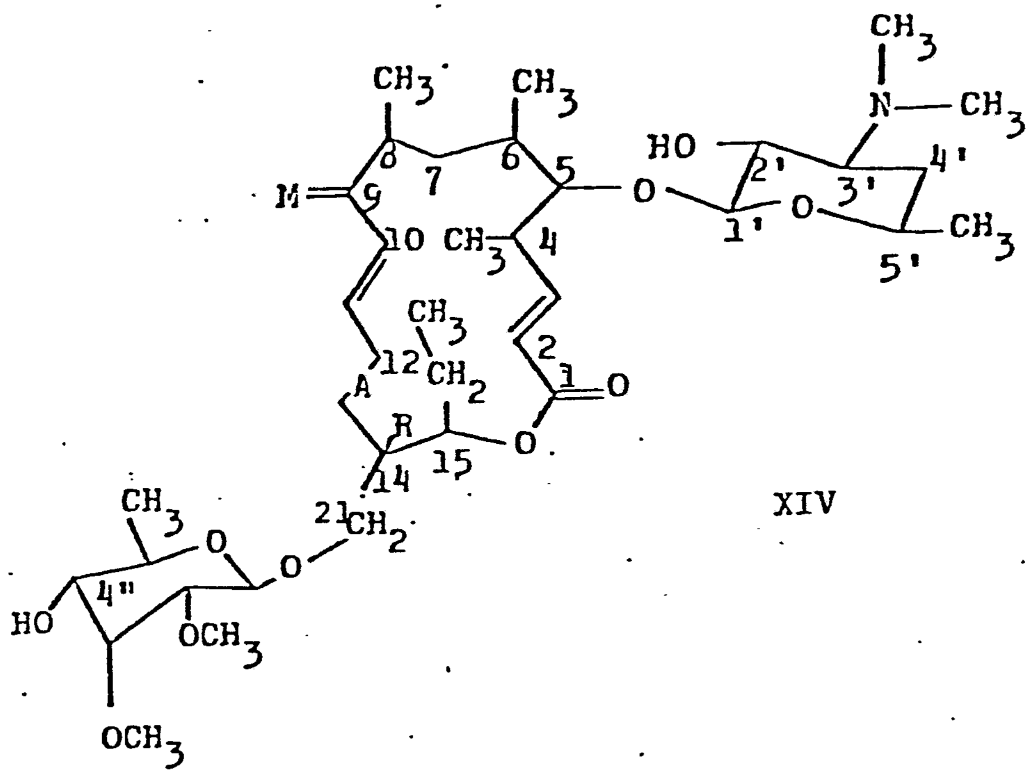

XIV

in the free form or in the form of an ester or salt, wherein R is hydrogen or hydroxy, M is oxo and A a double bond or together with the carbon atoms of positions 12 and 13 represents an oxirane ring, the free form compounds wherein A together with the carbon atoms of positions 12 and 13 represents an oxirane ring being designated antibiotic AR5-1 and antibiotic AR5-2.

4. A process according to claim 1, characterized in that a compound of formula I is prepared, wherein R is hydrogen or hydroxy, M is oxo or (H,OH), A and B represent double bonds and Z is $-CH_2OH$, $-CHO$, $CH_2NH_2$, $-CH_2NHR^2$, $CH_2NR^2R^3$, $-CH_2OCOR^2$, $-CH_2NHCOR^2$, $-CH_2NR^3COR^2$, $-CH_2NH-C(=NH)-R^2$ ———————————

$-CH_2NHC(=NH)-NHR^5$, $-CH_2NHCO-$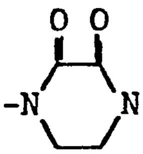$-CH_2CH_3$, $-CH_2O$-glycosyl excluding $-CH_2O$-mycinosyl and $-CH_2O$-(3"-desmethyl-mycinosyl), $-CH_2$- ureido or $-CH_2$—thioureido; and $R^2$, $R^3$ and $R^5$ are defined as in claim 1, preferably a compound, wherein M is (H,OH) and/or Z is $-CH_2OH$, ⊢—⊣ $-CH_2NH_2$, $-CH_2N(CH_3)_2$ or $-CH_2NHC(=NH)-NH_2$, in particular being desmycinosyl-12,13-desepoxy-12,13-dehydro antibiotic AR5-1.

5. A process according to claim 1, characterized in that a compound of formula XIV, set forth in claim 3 is prepared, wherein R is H or OH, M is (H,OH) and A is a double bond between the carbon atoms of positions 12 and 13 or together with the carbon atoms of postions 12 and 13 an oxirane ring.

6. A process according to claim 1, characterized in that a compound of formula I is prepared, wherein A together with the carbon atoms of positions 12 and 13 represents an oxirane ring; Z is the group of formula II,

wherein X is OH and Y is H, B together with the carbon atoms of positions 10 and 11 is the group $-CH_2-CH(Q^1)-$, wherein $-Q^1$ is $-NH_2$, $-NHC(O)R^6$, $-NHR^6$, $-NR^6R^7$,

-NH-C(O)-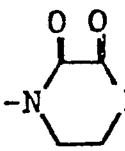$-CH_2CH_3$, an amino acid residue, an esterified amino acid residue or a heterocyclic radical of 6 members containing either nitrogen as the only heteroatom or nitrogen and a second heteroatom which is nitrogen, oxygen, or sulfur; and R, M, $R^6$ and $R^7$ are as defined in claim 1, in the free form or in the form of its ester, preferably a compound, wherein $Q^1$ is $-NH_2$, $-NHC(O)R^6$, thiomorpholino, 4-methylpiperazinyl, morpholino or 1-piperidinyl, in particular being 11-thiomorpholino-10,11-dihydro antibiotic AR5-2, 11-amino-10,11-dihydro antibiotic AR5-1, 11-amino-10,11-dihydro antibiotic AR5-2, 11-acetamido-10,11-dihydro antibiotic AR5-2, 11-(piperidinyl)-10,11-dihydro antibiotic AR5-2, 11-morpholino-10,11-dihydro antibiotic AR5-2, or 11-(4-methylpiperazinyl)-10,11-dihydro antibiotic AR5-2.

7. A process according to claim 1, characterized in that a compound of formula I is prepared, wherein A together with the carbon atoms of positions 12 and 13 represents an oxirane ring, Z is the group of general formula II in which X is OH and Y

is H, B together with the carbon atoms of positions 10 and 11 is the group $-CH_2-CH(Q^2)-$, wherein $-Q^2$ is $-S-R^6$, thioxanthyl, a sulfur containing amino acid residue, or a sulfur containing esterified amino acid residue; and R, M, and $R^6$ are as defined in claim 1, in the free form or in the form of its ester, preferably being 11-ethylthio-10,11-dihydro antibiotic AR5-1, 11-ethylthio-10,11-dihydro antibiotic AR5-2, 11-(p-methoxybenzylthio)-10,11-dihydro antibiotic AR5-2, or 11-L-cysteinyl-ethyl ester-10,11-dihydro antibiotic AR5-2.

8. A process according to claim 1, characterized in that a compound of formula I is prepared, wherein Z is the group of general formula II, wherein X is hydrogen, OH, mesyloxy, tosyloxy, trifluoromethanesulfonyloxy, halo, azido, amino, isocyanato, isothiocyanato, guanidino, $-NHR^6$, $-NR^6R^7$, $-NHR^8$, $-NHC(O)OR^6$, $-NHC(O)OR^8$, $-NHC(NH)R^6$, $-NH(CH_2)pOH$ or $-NH(CH_2)pOR^{10}$, wherein p is an integer of from 2 to 18, or $-NHCOR^6$ when Y is hydrogen; or Y in combination with X is oxo, $=N-NH_2$, $=N-NHR^{11}$ or $=NR^{11}$; A represents a double bond between the carbon atoms of positions 12 and 13 or, together with the carbon atoms of positions 12 and 13, an oxirane ring; B represents a double bond between the carbon atoms of positions 10 and 11; R, M, $R^6$, $R^7$, $R^8$, $R^{10}$ and $R^{11}$ are as defined in claim 1, preferably a compound, wherein X is hydrogen, OH, mesyloxy, halo, azido, amino, guanidino, $-NHR^6$, $-NR^6R^7$, $-NHR^8$, $-NHC(O)OR^6$, $-NHC(O)OR^8$ when Y is hydrogen; or Y in combination with X is oxo.

9. A process according to claim 8, characterized in that a compound is prepared, wherein X is mesyloxy and Y is hydrogen or Y in combination with X is oxo, in particular being 2'-acetyl-4"-dehydro antibiotic AR5-2, 2'-acetyl-4"-dehydro antibiotic AR5-1, 2'-acetyl-4"-methanesulfonyl-12,13-desepoxy-12,13-dehydro antibiotic AR5-2, or 4"-methanesulfonyl-12,13-desepoxy-12,13-dehydro antibiotic AR5-2 being prepared.

10. A process according to claim 1, characterized in that 10,11-dihydro antibiotic AR5-1 is prepared.

11. Process for the preparation of gentamicin C complex characterized by the incubation of a microorganism of the species Micromonospora polytrota in particular Micromonospora polytrota NRRL 12066 in an aqueous nutrient medium under aerobic conditions and isolation from the broth.

European Patent Office

# EUROPEAN SEARCH REPORT

Application number
EP 80 81 0346

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.³) |
|---|---|---|---|
| | JOURNAL OF ANTIBIOTICS, vol. XXX, no. 8, August 1977, 673, 674.<br>SUPAPONG BHUWAPATHANAPUN et al.: "High pressure liquid chromatography of the macrolide antibiotic tylosin".<br>* Page 673; figure 1, Tylosin B *<br>-- | 1,2,15 | C 07 H 17/08<br>15/22<br>A 61 K 31/70<br>C 12 P 19/62<br>19/48//<br>(C 12 P 19/62<br>C 12 R 1/29)<br>(C 12 P 19/48<br>C 12 R 1/29) |
| DPX | GB - A - 2 020 647 (TOYO JOZO CO.)<br>* Whole document *<br>& FR - A - 2 422 544<br>& DE - A - 2 918 711<br>& NL - A - 79 03601<br>-- | 1-12, 14-15 | TECHNICAL FIELDS SEARCHED (Int.Cl.³) |
| P | JOURNAL OF ANTIBIOTICS, vol. 33, no. 4, April 1980, pages 364-376, Tokyo, JP<br>SHUZO SATOI et al. "Mycinamicins, new macrolide antibiotics. I. Taxonomy, production, isolation, characterization and properties".<br>* Whole document *<br>---- | 1,2,15 | C 07 H 17/08<br>A 61 K 31/70<br>C 12 P 19/62<br>19/48<br>C 12 R 1/29<br>C 07 H 17/04<br>15/22<br>C 12 P 19/44<br>C 12 P 19/46<br>C 12 P 19/60 |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 26-03-1981 | VERHULST |

European Patent Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## ☒ LACK OF UNITY OF INVENTION

The Search Division considers that the present European patent application does not comply with the requirement of unity of invention and relates to several inventions or groups of inventions,

namely:

1. Claims 1-3,5,8,11-15: Fermentative production of antibiotics, antibiotics corresponding to formula XIV of claim 2 and pharmaceutical compositions containing them.
2. Claims 1,4,6-10,11,14,15: Chemical process for the preparation of macrolide antibiotics, chemically synthesised macrolide compounds corresponding to formula I of claim 1 and pharmaceutical compositions containing them.
3. Claims 1,8-11: Intermediates per se.

☒ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☐ None of the further search fees has been paid within the fixed time limit. The present European search report has been drawn up for those parts of the European patent application which relate to the invention first mentioned in the claims,

namely claims: